# EUROPEAN PATENT APPLICATION

(11) **EP 4 600 245 A1**
(43) Date of publication of application: **13.08.2025**
(21) Application number: 23890643.2
(22) Date of filing: 04.11.2023
(51) Int. Cl.: C07D 307/91, C07D 209/86, C07D 309/04, C07D 307/14, C07C 211/61, C09K 11/06

(54) **SPIRO COMPOUND COMPRISING ASYMMETRIC ALKYL SUBSTITUTION AND ORGANIC LIGHT-EMITTING DEVICE**

(30) Priority: 14.11.2022 CN 202211416912; 24.10.2023 CN 202311379348
(71) Applicant: Guangdong Aglaia Optoelectronic Materials Co., Ltd, Foshan, Guangdong 528300 (CN)
(72) Inventor: CHEN, Shaofu, Foshan, Guangdong 528300 (CN); DAI, Lei, Foshan, Guangdong 528300 (CN); CAI, Lifei, Foshan, Guangdong 528300 (CN)
(74) Representative: Bals & Vogel Patentanwälte PartGmbB
(86) International application number: PCT/CN2023/129818
(87) International publication number: WO 2024/104207

(57) **Abstract**

Provided are a spiro compound comprising an asymmetric alkyl substitution and an organic light-emitting device. The spiro compound has a structure represented by formula (1). The material has advantages such as high optical and electrical stability, a low sublimation temperature, a low driving voltage, small lateral mobility of charge carriers, high light-emitting efficiency, long device service life, and can be used in the organic light-emitting device. Meanwhile, the material has a relatively low melting point and is beneficial to material evaporation stability as a molten material. The compound is particularly used as a hole injection and transmission material, and has the possibility of being applied in the AMOLED industry.

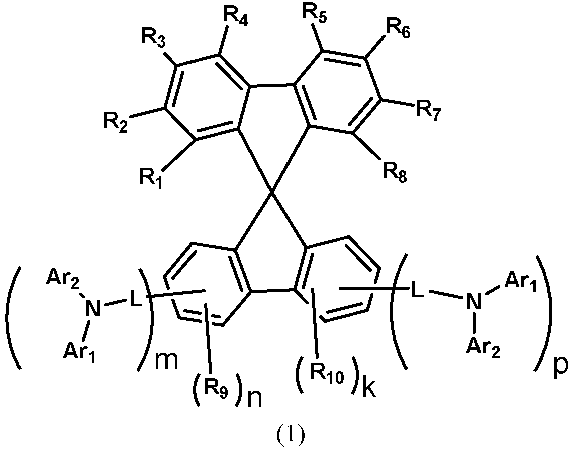

## Description

### TECHNICAL FIELD

The present disclosure relates to the technical field of organic electroluminescence, especially to an organic light-emitting material suitable for organic electroluminescent devices, particularly to a spiro compound comprising an asymmetric alkyl substitution and an organic electroluminescent device.

### BACKGROUND

Currently, organic electroluminescent devices (OLEDs), as a new generation of display technology, have gained increasing attention in both display and lighting technology, with very broad application prospects. However, compared to market application requirements, the luminous efficiency, driving voltage, and lifespan of OLED devices still need to be further enhanced and improved.

Generally, the basic structure of an OLED device consists of various organic functional material thin films sandwiched between metal electrodes, forming a sandwich-like structure. Driven by an electric current, holes and electrons are injected from the cathode and anode, respectively. After moving a certain distance, the holes and electrons recombine in the emitting layer and are released in the form of light or heat, resulting in the luminescence of the OLED. However, organic functional materials are the core components of organic electroluminescent devices. The thermal stability, photochemical stability, electrochemical stability, quantum yield, film formation stability, crystallinity, color saturation, and other properties of the materials are all major factors affecting the performance of the devices.

In order to obtain organic light-emitting devices with excellent performance, the selection of materials is particularly important. This includes not only emissive materials responsible for light emission, but also functional materials such as hole injection materials, hole transport materials, host materials, electron transport materials, and electron injection materials that mainly function for carrier injection and transport in the device. The selection and optimization of these materials can improve the transport efficiency of holes and electrons, allowing the balance of holes and electrons in the device, thereby improving the device voltage, luminous efficiency, and lifespan.

Patent document 1 (CN103108859B) discloses the structure of spirofluorene arylamine 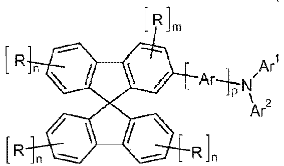 used as a hole transport material demonstrating improved device performance, but the device lifetime, especially the lifetime of blue light emitting devices still needs further improvement. In addition, the lateral hole mobility of such material also needs to be further improved to provide better low grayscale color purity for OLED products. Patent document 2 (CN103641726B) discloses the structure of spirofluorene arylamine 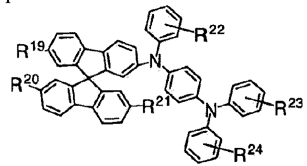 used as a second hole transport material, however, the device performance of such material needs to be greatly improved, especially in terms of device efficiency. Patent document 3 (CN111548278A) discloses that the arylamine moiety of the spirofluorene arylamine 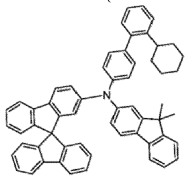 contains substituents such as alkyl, deuterium, or cycloalkyl used as hole transport materials. The performance of devices using such materials also needs to be further improved, especially in terms of device lifespan. In the non-patent literature 1 (J. Mater. Chem., 2005, 15, 2455-2463), Jiun YiShen et al. discloses a class of spirofluorene-based blue light-emitting materials, such as 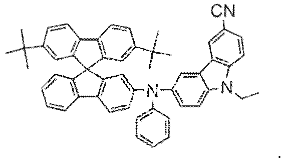 When used in the blue light-emitting layer, these materials require improvements in the luminous efficiency and lifetime of the device. Additionally, when used as hole transport materials, these issues also need to be optimized and improved. Patent document 4 (CN114835591A) discloses 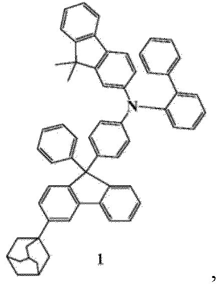 the arylamine hole transport material containing mono-cycloalkyl-substituted diphenylfluorene. The device performance of such materials still needs to be further improved, especially in terms of device efficiency. Additionally, the reported compounds have a high melting point, making them unsuitable for industrial applications as molten materials.

### SUMMARY

To address the above-mentioned deficiencies, the present disclosure provides an organic electroluminescent device with high-performance and a spiro compound material comprising an asymmetric alkyl substitution capable of realizing such an organic electroluminescent device.

In accordance with the present disclosure, the spiro compound comprising an asymmetric alkyl substitution has a structure of formula (1). The spiro compound comprising an asymmetric alkyl substitution provided herein has advantages such as high photoelectric stability, low sublimation temperature, low driving voltage, low lateral carrier mobility, high luminous efficiency and long device service life, making it suitable for use in organic electroluminescent devices. Additionally, the compound has a lower melting point, which is beneficial for evaporation stability of the material as a melt-processable material. The compound, particularly as a hole injection and transport material, has potential applications in the AMOLED industry.

Provided is a spiro compound comprising an asymmetric alkyl substitution, having a structure of formula (1), 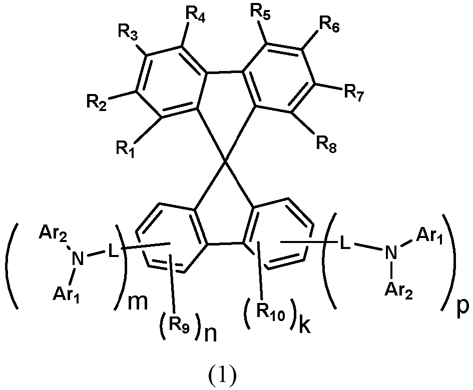
where R₁-R₁₀ are each independently selected from a group consisting of hydrogen, deuterium, halogen, cyano, hydroxy, thiol, amino, a substituted or unsubstituted C1-C10 alkyl, a substituted or unsubstituted C1-C10 heteroalkyl, a substituted or unsubstituted C3-C20 cycloalkyl, a substituted or unsubstituted C3-C20 heterocycloalkyl, a substituted or unsubstituted C2-C10 alkenyl, a substituted or unsubstituted C2-C10 alkynyl, a substituted or unsubstituted C6-C30 aryl, a substituted or unsubstituted C2-C30 heteroaryl, a substituted or unsubstituted (tri-C1-C10 alkyl)silyl, a substituted or unsubstituted (tri-C6-C12 aryl)silyl, a substituted or unsubstituted (di-C1-C10 alkyl)(mono-C6-C30 aryl)silyl, or a substituted or unsubstituted (mono-C1-C10 alkyl)(di-C6-C30 aryl)silyl; alternatively, two adjacent groups among R₁-R₈ and R₉-R₁₀ are connected to each other to form an aliphatic or aromatic ring structure;
where at least one of R₅-R₈ is a substituted or unsubstituted C1-C10 alkyl, or a substituted or unsubstituted C1-C10 heteroalkyl; and at least one of R₁-R₄ is a substituted or unsubstituted C3-C20 cycloalkyl, or a substituted or unsubstituted C3-C20 heterocycloalkyl;
where L is independently selected from a group consisting of a single bond, a substituted or unsubstituted C6-C30 arylene, or a substituted or unsubstituted C2-C30 heteroarylene;
where Ar1 and Ar2 are each independently selected from a substituted or unsubstituted C6-C30 aryl, or a substituted or unsubstituted C2-C30 heteroaryl;
where m, n, k and p are each independently selected from 0 or an integer from 1 to 4, with the proviso that m + n = 4 and p + k = 4;
where the heteroalkyl, the heterocycloalkyl, the heteroarylene, and the heteroaryl each comprises at least one heteroatom selected from O, N, Si, or S; and
where the substitution refers to being substituted with deuterium, halogen, C1-C6 alkyl, C3-C6 cycloalkyl, C1-C6 alkyl-substituted amino, cyano, isocyano or phosphino, with the number of substitutions being from a monosubstitution to a maximum substitution.

That two adjacent groups among R₁-R₈ and R₉-R₁₀ are connected to each other to form an aliphatic or aromatic ring structure refers to that the groups at adjacent positions are bonded to each other to form an aliphatic or aromatic ring structure with carbon atoms of the aromatic ring where they are located, or that R₄ and R₅ are bonded to each other, and R₉ and R₁₀ are bonded to each other, to form an aliphatic or aromatic ring structure with the atoms on the aromatic ring where they are located.

In some embodiments, in the spiro compound, m + p = 1.

In some embodiments, the spiro compound has a structure represented by formulas (2) to (12), 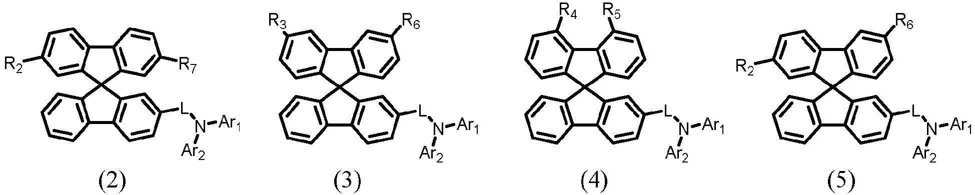 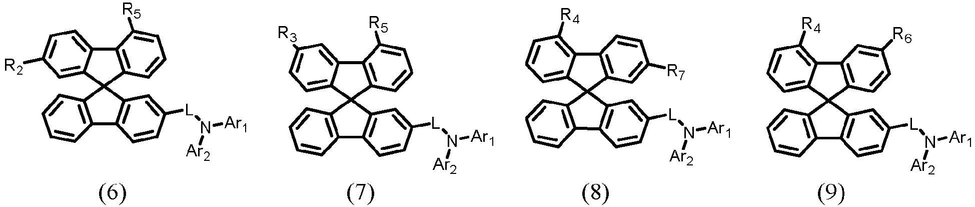 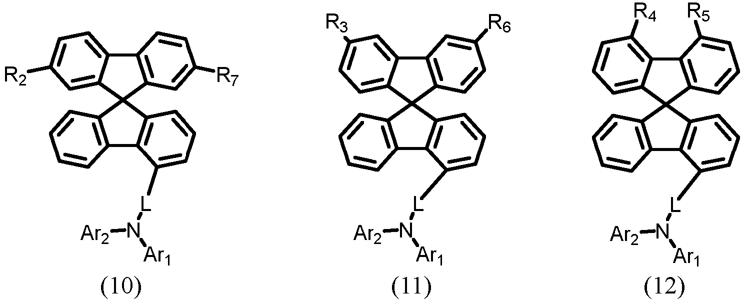
where each of R₅, R₆ and R₇ is a substituted or unsubstituted C1-C10 alkyl, or a substituted or unsubstituted C1-C10 heteroalkyl; and each of R₂, R₃ and R₄ is a substituted or unsubstituted C3-C20 cycloalkyl, or a substituted or unsubstituted C3-C20 heterocycloalkyl; and
L, Ar1, and Ar2 are as defined above.

In some embodiments, the spiro compound has a structure represented by formulas (2) to (12), where the L is a single bond.

In some embodiments, the spiro compound has a structure represented by formulas (2) and (10).

In some embodiments, the spiro compound has a structure represented by formulas (13) to (14): 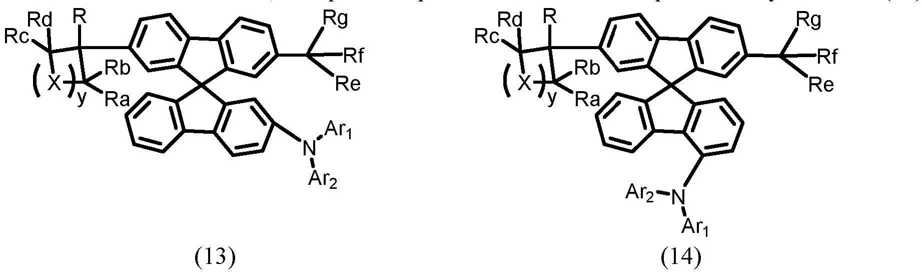
where X is independently selected from C(R₀)₂, Si(R₀)₂, O, S or NR₀;
where y is independently 0 or an integer from 1 to 7; and a three-membered ring is formed when y=0; each X is same or different when y≥2;
where R, R₀, and Ra-Rd are each independently selected from a group consisting of hydrogen, deuterium, halogen, cyano, isocyano, phosphino, amino, a substituted or unsubstituted C1-C10 alkyl, a substituted or unsubstituted C1-C10 heteroalkyl, and a substituted or unsubstituted C3-C20 cycloalkyl; alternatively, any two of Ra, Rb, Rc, Rd, and/or any two R₀ groups, and/or R and one of Ra, Rb, Rc, Rd, R₀, are interconnected to form a ring structure; and Re-Rg are each independently selected from a group consisting of hydrogen, deuterium, halogen, C1-C6 alkyl, C1-C6 alkyl-substituted amino, cyano, isocyano, or phosphino; and
the substitution refers to being substituted with deuterium, F, Cl, Br, C1-C6 alkyl, C3-C6 cycloalkyl, C1-C6 alkyl-substituted amino, cyano, isocyano, or phosphino, with the number of substitutions being from a monosubstitution to a maximum substitution.

That any two of Ra, Rb, Rc, Rd, and/or any two R₀ groups, and/or R and one of Ra, Rb, Rc, Rd and R₀ are connected to each other to form an aliphatic or aromatic ring structure refers to that any two of Ra, Rb, Rc, and Rd, and/or two R₀ groups, and/or R and one of Ra, Rb, Rc, Rd, and R₀ are bonded to each other to form a ring structure with the atoms on the aliphatic ring where they are located.

In some embodiments, the spiro compound is one where R is hydrogen, deuterium, a substituted or unsubstituted C1-C10 alkyl, or a substituted or unsubstituted C1-C10 heteroalkyl.

In some embodiments, the spiro compound is one where y is a number greater than or equal to 2.

In some embodiments, the spiro compound is one where at most one X group in two or more X groups is selected from O, S, or NR₀.

In some embodiments, the spiro compound is one where two R₀ groups and/or R and R₀ are interconnected to form a ring structure.

In some embodiments, the spiro compound is one where Ar1 and Ar2 are different, and Ar1 and Ar2 are each independently selected from a group consisting of a substituted or unsubstituted phenyl, a substituted or unsubstituted biphenyl, a substituted or unsubstituted naphthyl, a substituted or unsubstituted fluorenyl, a substituted or unsubstituted dibenzofuranyl, or a substituted or unsubstituted carbazolyl; and where the substitution on the phenyl, biphenyl, naphthyl, fluorenyl, dibenzofuranyl, and carbazolyl refers to being substituted with deuterium, F, Cl, Br, C6-C10 aryl, C5-C12 heteroaryl, C1-C6 alkyl, or C3-C6 cycloalkyl; and the heteroaryl comprises at least one heteroatom selected from O, N, or S.

In some embodiments, the spiro compound is represented by one of the following structural formulas, or being correspondingly partially or completely deuterated or fluorinated:

| | | | |
|---|---|---|---|
| 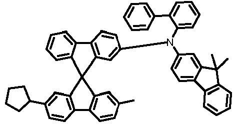 | 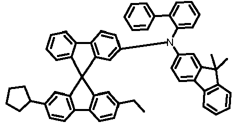 | 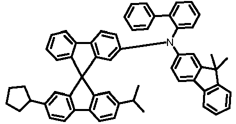 | 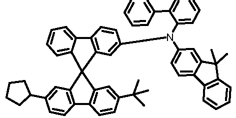 |
| CPD001 | CPD002 | CPD003 | CPD004 |
| 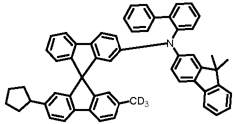 | 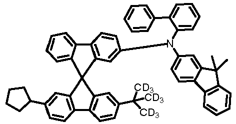 | 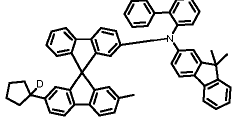 | 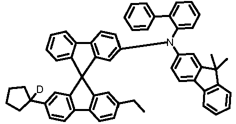 |
| CPD005 | CPD006 | CPD007 | CPD008 |
| 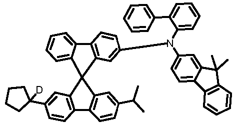 | 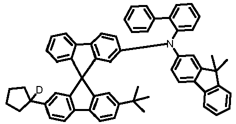 | 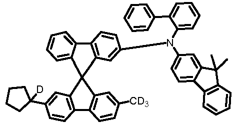 | 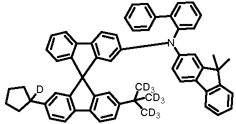 |
| CPD009 | CPD010 | CPD011 | CPD012 |
| 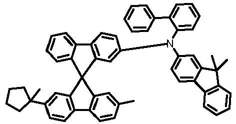 | 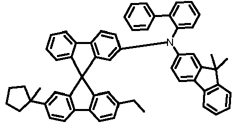 | 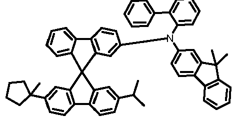 | 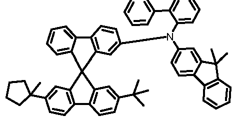 |
| CPD013 | CPD014 | CPD015 | CPD016 |
| 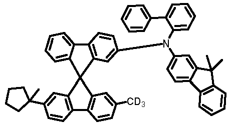 | 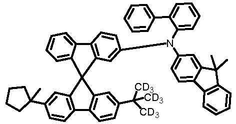 | 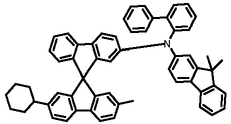 | 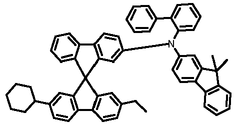 |
| CPD017 | CPD018 | CPD019 | CPD020 |
| 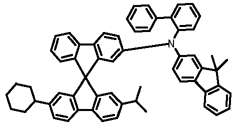 | 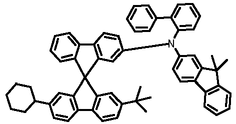 | 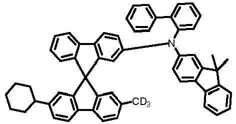 | 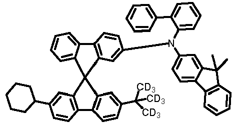 |
| CPD021 | CPD022 | CPD023 | CPD024 |
| 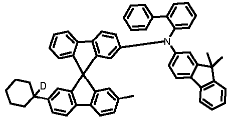 | 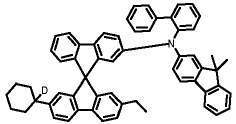 | 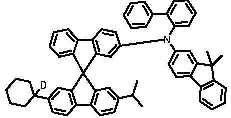 | 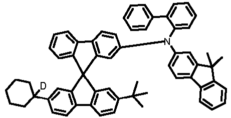 |
| CPD025 | CPD026 | CPD027 | CPD028 |
| 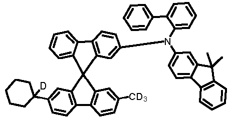 | 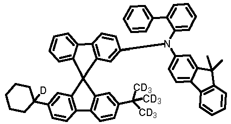 | 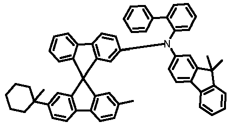 | 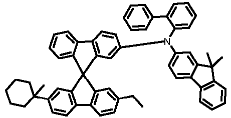 |
| CPD029 | CPD030 | CPD031 | CPD032 |
| 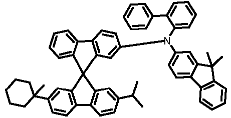 | 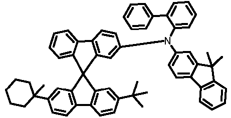 | 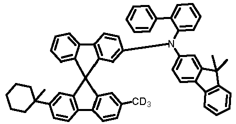 | 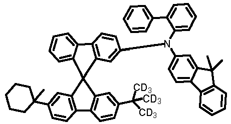 |
| CPD033 | CPD034 | CPD035 | CPD036 |
| 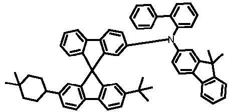 | 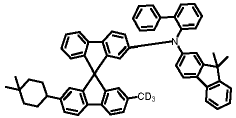 | 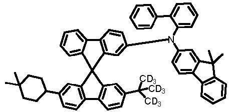 | 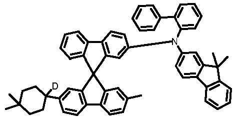 |
| CPD037 | CPD038 | CPD039 | CPD040 |
| 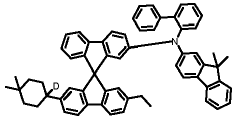 | 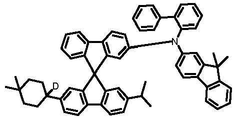 | 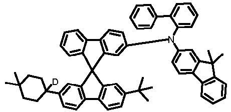 | 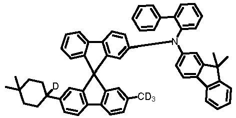 |
| CPD041 | CPD042 | CPD043 | CPD044 |
| 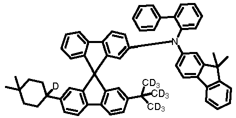 | 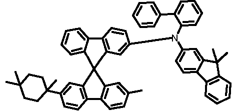 | 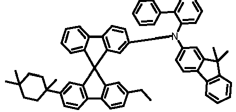 | 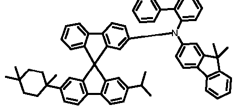 |
| CPD045 | CPD046 | CPD047 | CPD048 |
| 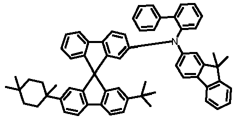 | 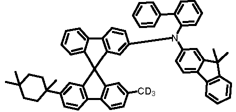 | 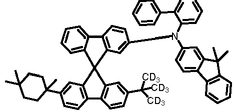 | 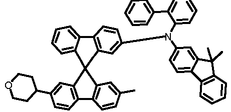 |
| CPD049 | CPD050 | CPD051 | CPD052 |
| 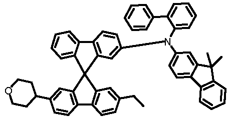 | 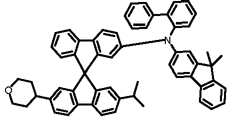 | 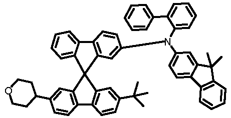 | 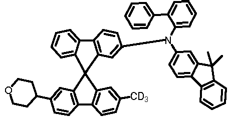 |
| CPD053 | CPD054 | CPD055 | CPD056 |
| 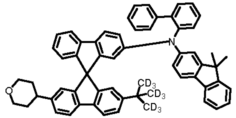 | 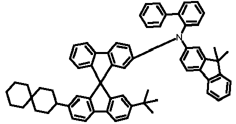 | 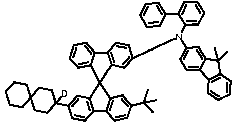 | 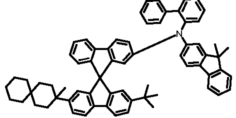 |
| CPD057 | CPD058 | CPD059 | CPD060 |
| 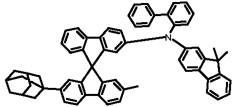 | 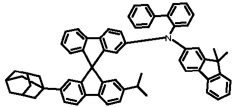 | 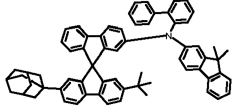 | 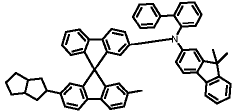 |
| CPD061 | CPD062 | CPD063 | CPD064 |
| 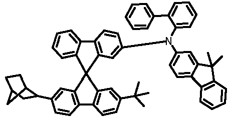 | 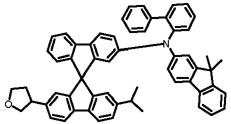 | 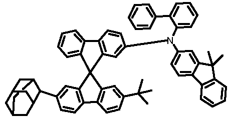 | 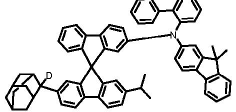 |
| CPD065 | CPD066 | CPD067 | CPD068 |
| 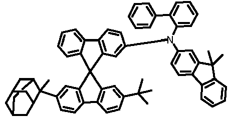 | 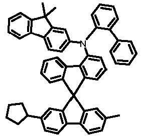 | 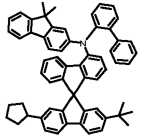 | 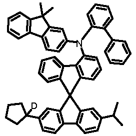 |
| CPD069 | CPD070 | CPD071 | CPD072 |
| 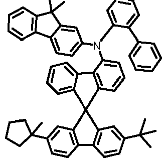 | 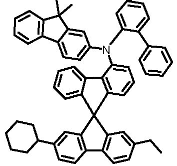 | 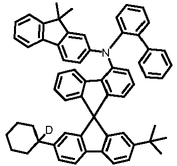 | 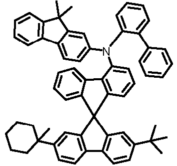 |
| CPD073 | CPD074 | CPD075 | CPD076 |
| 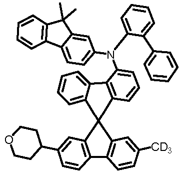 | 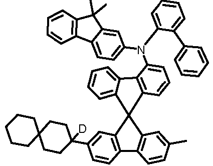 | 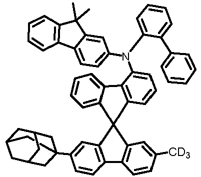 | 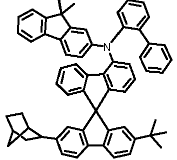 |
| CPD077 | CPD078 | CPD079 | CPD080 |
| 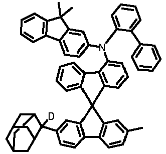 | 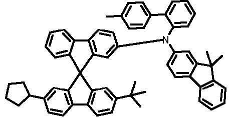 | 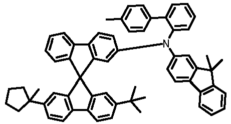 | 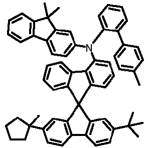 |
| CPD081 | CPD082 | CPD083 | CPD084 |
| 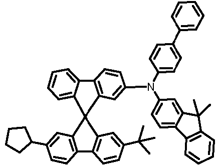 | 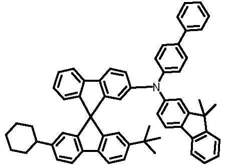 | 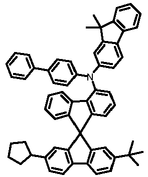 | 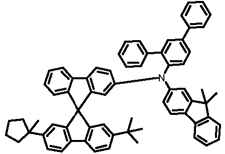 |
| CPD085 | CPD086 | CPD087 | CPD088 |
| 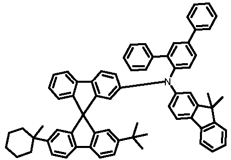 | 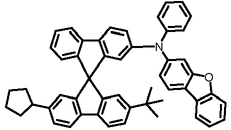 | 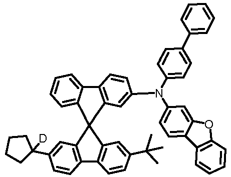 | 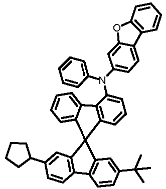 |
| CPD089 | CPD090 | CPD091 | CPD092 |
| 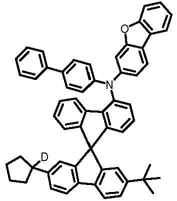 | 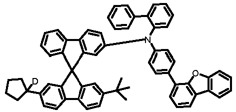 | 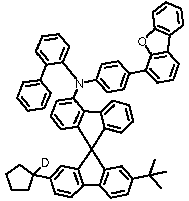 | 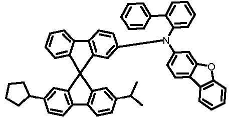 |
| CPD093 | CPD094 | CPD095 | CPD096 |
| 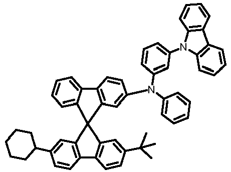 | 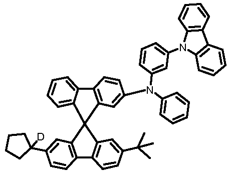 | 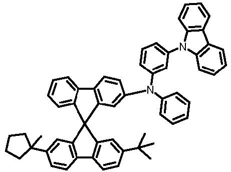 | 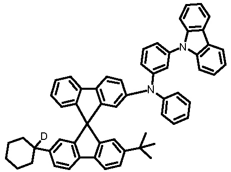 |
| CPD097 | CPD098 | CPD099 | CPD 100 |
| 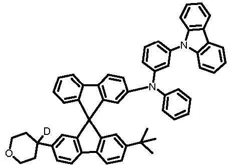 | 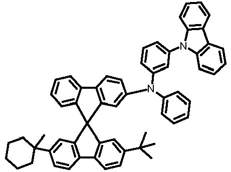 | 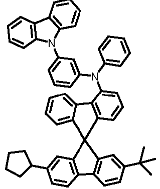 | 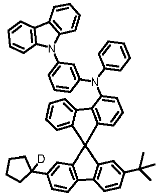 |
| CPD101 | CPD 02 | CPD103 | CPD104 |
| 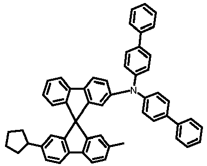 | 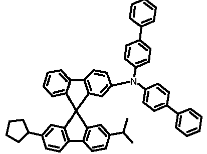 | 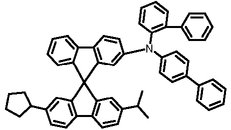 | 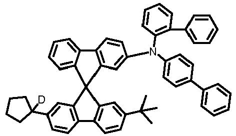 |
| CPD105 | CPD106 | CPD107 | CPD108 |
| 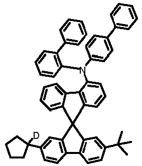 | 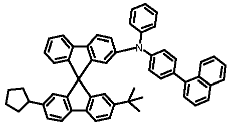 | 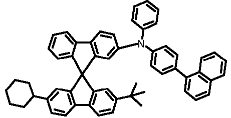 | 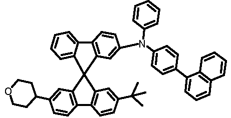 |
| CPD109 | CPD110 | CPD111 | CPD112 |
| 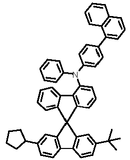 | 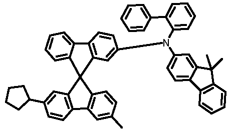 | 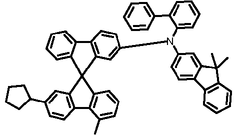 | 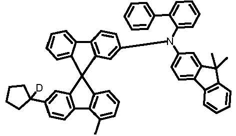 |
| CPD113 | CPD114 | CPD115 | CPD116 |
| 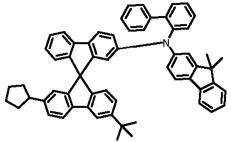 | 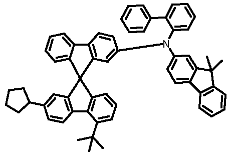 | 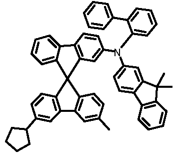 | 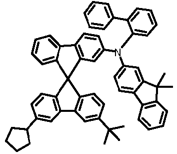 |
| CPD117 | CPD118 | CPD119 | CPD120 |
| 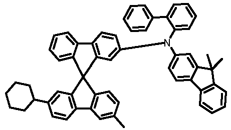 | 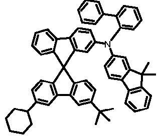 | 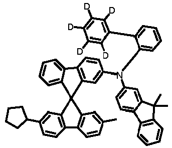 | 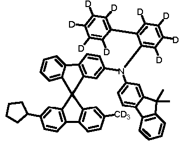 |
| CPD121 | CPD122 | CPD123 | CPD124 |
| 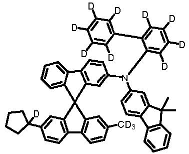 | 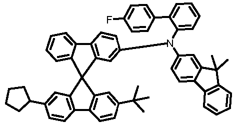 | 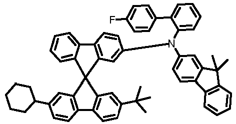 | 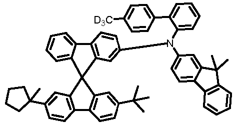 |
| CPD125 | CPD126 | CPD127 | CPD128 |

Provided is an organic electroluminescent device comprising the above-mentioned spiro compound, where the spiro compound is a material for a hole injection layer and/or a hole transport layer of the organic electroluminescent device.

The materials in the present disclosure have the advantages such as high photoelectric stability, low sublimation temperature, low driving voltage, low carrier lateral mobility, high luminous efficiency, and long device service life, making it suitable for use in organic electroluminescent devices. The material particularly serves as hole injection and transport materials, with potential applications in the AMOLED industry.

### BRIEF DESCRIPTION OF THE DRAWINGS

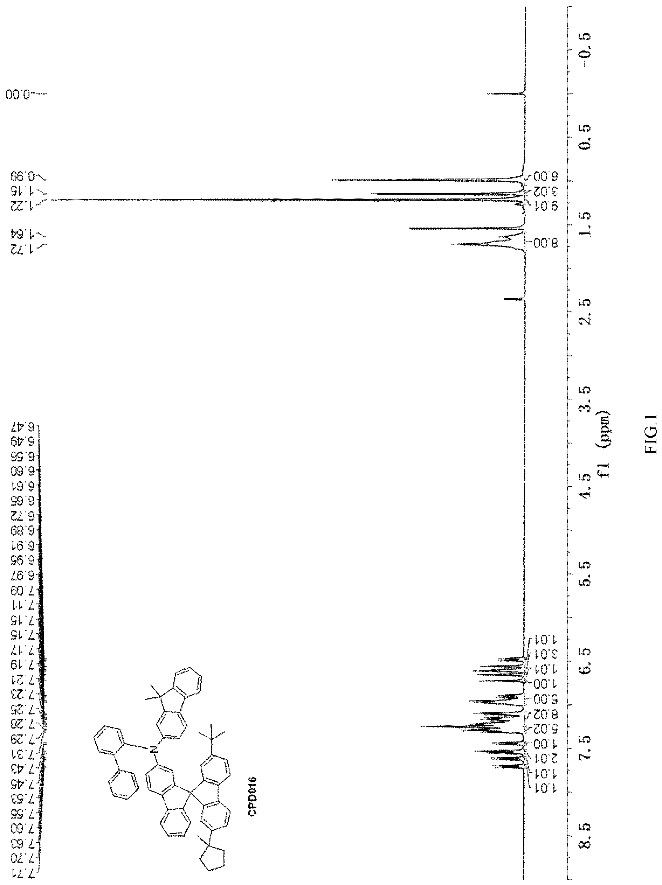

Fig. 1 shows a ¹H NMR spectrum of compound CPD016; and
Fig. 2 shows a schematic diagram of a device structure in the present disclosure.

### DETAILED DESCRIPTION

The compound in the present disclosure is a spiro compound comprising an asymmetric alkyl substitution, which has a structure of formula (1), 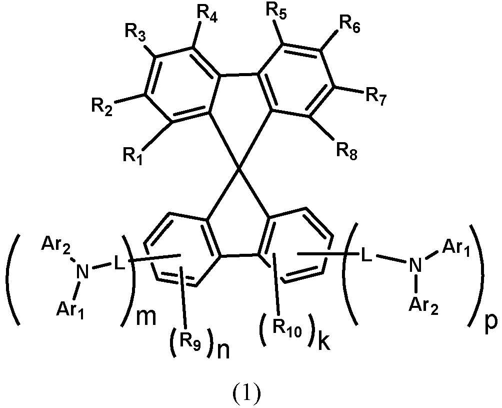
where R₁-R₁₀ are each independently selected from a group consisting of hydrogen, deuterium, halogen, cyano, hydroxy, thiol, amino, a substituted or unsubstituted C1-C10 alkyl, a substituted or unsubstituted C1-C10 heteroalkyl, a substituted or unsubstituted C3-C20 cycloalkyl, a substituted or unsubstituted C3-C20 heterocycloalkyl, a substituted or unsubstituted C2-C10 alkenyl, a substituted or unsubstituted C2-C10 alkynyl, a substituted or unsubstituted C6-C30 aryl, a substituted or unsubstituted C2-C30 heteroaryl, a substituted or unsubstituted (tri-C1-C10 alkyl)silyl, a substituted or unsubstituted (tri-C6-C12 aryl)silyl, a substituted or unsubstituted (di-C1-C10 alkyl)(mono-C6-C30 aryl)silyl, or a substituted or unsubstituted (mono-C1-C10 alkyl)(di-C6-C30 aryl)silyl; alternatively, two adjacent groups among R₁-R₈ and R₉-R₁₀ are connected to each other to form an aliphatic or aromatic ring structure;
where at least one of R₅-R₈ is a substituted or unsubstituted C1-C10 alkyl, or a substituted or unsubstituted C1-C10 heteroalkyl; and at least one of R₁-R₄ is a substituted or unsubstituted C3-C20 cycloalkyl, or a substituted or unsubstituted C3-C20 heterocycloalkyl;
where L is independently selected from a group consisting of a single bond, a substituted or unsubstituted C6-C30 arylene, or a substituted or unsubstituted C2-C30 heteroarylene;
where Ar1 and Ar2 are each independently selected from a substituted or unsubstituted C6-C30 aryl, or a substituted or unsubstituted C2-C30 heteroaryl;
where m, n, k and p are each independently selected from 0 or an integer from 1 to 4, with the proviso that m + n = 4 and p + k = 4; and
where the heteroalkyl, the heterocycloalkyl, the heteroarylene, and the heteroaryl each comprises at least one heteroatom selected from O, N, Si, or S.

Examples of each group in the compound represented by formula (1) are illustrated below.

It should be noted that in the Specification, the term "a carbon number of a to b" in the expression "a substituted or unsubstituted X group having a carbon number of a to b" refers to the carbon number of the X group when it is unsubstituted, excluding the carbon number of the substituent when the X group is substituted.

C1-C10 alkyl is a linear or branched alkyl, specifically including methyl, ethyl, propyl, isopropyl, n-butyl, isobutyl, sec-butyl, tert-butyl, n-pentyl and its isomers, n-hexyl and its isomers, n-heptyl and its isomers, n-octyl and its isomers, n-nonyl and its isomers, or n-decyl and its isomers, etc. In some embodiments, the alkyl is methyl, ethyl, propyl, isopropyl, n-butyl, isobutyl, sec-butyl, or tert-butyl. More specifically, in some embodiments, it is propyl, isopropyl, isobutyl, sec-butyl, or tert-butyl.

Examples of C3-C20 cycloalkyl include cyclopropyl, cyclobutyl, cyclopentyl, cyclohexyl, 1-adamantyl, 2-adamantyl, 1-norbornyl, or 2-norbornyl, etc. In some embodiments, the cycloalkyl is cyclopentyl or cyclohexyl.

Examples of C2-C10 alkenyl include vinyl, propenyl, allyl, 1-butadienyl, 2-butadienyl, 1-hexatrienyl, 2-hexatrienyl, or 3-hexatrienyl, etc. In some embodiments, the alkenyl is propenyl or allyl.

The term "C1-C10 heteroalkyl" refers to linear or branched alkyl or cycloalkyl groups composed of atoms other than carbon and hydrogen. Representative examples include, but are not limited to: mercaptomethyl, methoxymethyl, ethoxymethyl, tert-butoxymethyl, N,N-dimethylalkyl, epoxybutyl, epoxypentyl, epoxyhexyl, etc. In some embodiments, the heteroalkyl group is selected from methoxymethyl and epoxypentyl.

Specific examples of aryl include phenyl, naphthyl, anthryl, phenanthrenyl, tetracenyl, pyrenyl, chrysenyl, benzo[c]phenanthrenyl, benzo[g]chrysenyl, fluorenyl, benzofluorenyl, dibenzofluorenyl, biphenylyl, terphenylyl, quaterphenylyl, or fluoranthenyl, etc. In some embodiments, the aryl is phenyl or naphthyl.

Specific examples of heteroaryl include pyrrolyl, pyrazinyl, pyridyl, pyrimidinyl, triazinyl, indolyl, isoindolyl, imidazolyl, furyl, benzofuranyl, isobenzofuranyl, dibenzofuranyl, dibenzothienyl, aza-dibenzofuranyl, aza-dibenzothienyl, diaza-dibenzofuranyl, diaza-dibenzothienyl, quinolinyl, isoquinolinyl, quinoxalinyl, carbazolyl, phenanthridinyl, acridinyl, phenanthrolinyl, phenazinyl, phenothiazinyl, phenoxazinyl, oxazolinyl, oxadiazolyl, furazanyl, thienyl, benzothienyl, dihydroacridinyl, aza-carbazolyl, diaza-carbazolyl, quinazolinyl, etc. In some embodiments, the heteroaryl is pyridyl, pyrimidinyl, triazinyl, dibenzofuranyl, dibenzothienyl, aza-dibenzofuranyl, aza-dibenzothienyl, diaza-dibenzofuranyl, diaza-dibenzothienyl, carbazolyl, aza-carbazolyl, or diaza-carbazolyl.

The following embodiments are merely to facilitate understanding of the technical invention and should not be regarded as specific limitations of the present disclosure.

Raw materials and solvents involved in the synthesis of the compounds in the present disclosure are purchased from Alfa, Acros and other suppliers well known to those skilled in the art.

### Example

### Synthesis of compound CPD001

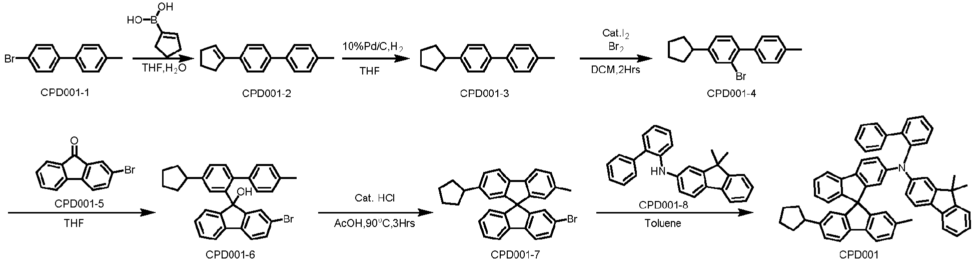

### Synthesis of compound CPD001-2

Compound **CPD001-1** (20.00 g, 80.93 mmol), cyclopentene-1-ylboronic acid (10.87 g, 97.11 mmol), bis(4-dimethylaminophenyl)di-tert-butylphosphine palladium dichloride (0.57 g, 0.81 mmol), potassium carbonate (16.78 g, 121.40 mmol), tetrahydrofuran (200 mL), and deionized water (60 mL) were added to a 1,000 mL three-necked round-bottom flask. The flask was purged with nitrogen three times and heated to 66°C for overnight reaction. The consumption of the starting material **CPD001-1** was monitored by TLC (n-hexane as a developing solvent). After the reaction was completed, the reaction mixture was cooled to room temperature. Deionized water (200 mL) and methanol (200 mL) were added, and the mixture was stirred at room temperature for 2 h. The mixture was filtered by suction to obtain a solid, and the solid was rinsed with a mixed solvent of methanol and water (200 mL). After vacuum drying at 80°C for 6 h, a gray solid compound **CPD001-2** (18.02 g, purity: 99.52%, yield: 95.04%) was obtained, mass spectrometry: 235.24 (M+H).

### Synthesis of compound CPD001-3

Compound **CPD001-2** (18.00 g, 76.81 mmol) and tetrahydrofuran (180 mL) were added to a 500 mL single-necked round-bottom flask, followed by the addition of 10% (mass fraction) palladium on carbon (1.8 g). The flask was purged with hydrogen four times, and the reaction mixture was stirred at room temperature for overnight reaction for 24 h. The consumption of the starting material **CPD001-2** was monitored by **TLC** (n-hexane as the developing solvent).

The reaction solution was directly filtered through 200-300 mesh silica gel, and the silica gel was washed with 500 mL of dichloromethane until the filter cake showed no obvious fluorescence. The silica gel column chromatography (200-300 mesh silica gel, hexane as an eluent) was then performed. After elution, a white solid compound **CPD001-3** (17.72 g, purity: 99.92%, yield: 97.63%) was obtained by vacuum concentration at 65°C, mass spectrometry: 237.14 (M+H).

### Synthesis of compound CPD001-4

**CPD001-3** (17.00 g, 71.92 mmol) and dichloromethane (250 mL) were added into a 500 mL three-necked round-bottom flask. The system was then cooled to -5°C, followed by the addition of elemental iodine (1.09 g, 3.60 mmol). Bromine (12.64 g, 79.12 mmol) was slowly added dropwise over 10 min, and then the reaction was maintained at -5°C for 2 h. The consumption of the starting material **CPD001-3** was monitored by TLC (n-hexane as the developing solvent). Once the reaction was complete, the reaction was stopped. The reaction was quenched with a saturated aqueous sodium thiosulfate solution until the potassium iodide-starch test paper no longer turned blue. The layers were separated, and the organic phase was washed with deionized water (3×100 mL). Silica gel column chromatography was performed (200-300 mesh silica gel, n-hexane as the eluent). After elution, a yellow liquid compound **CPD001-4** (20.66 g, purity: 99.86%, yield: 91.11%) was obtained by vacuum concentration at 65°C, mass spectrometry: 315.08 (M+H).

### Synthesis of compound CPD001-6

**CPD001-4** (20.00 g, 63.44 mmol) and dried tetrahydrofuran (200 mL) were added to a 500 mL three-necked round-bottom flask. The flask was purged with nitrogen three times. The system was then cooled to -78°C. A 2.5 mol/L solution of n-butyllithium in n-hexane (27.90 mL, 69.78 mmol) was added dropwise over 10 min, and the reaction was maintained at -78°C for 1 h. Solid **CPD001-**5 (18.08 g, 69.78 mmol) was directly added over 3 min, and the system was heated to -30°C, turning brown-red. The reaction was carried out for 3 h. The reaction was monitored by TLC (ethyl acetate: n-hexane = 1:50 as the developing solvent) until both starting materials, **CPD00 -4** and **CPD001-5,** were completely consumed. The reaction was quenched by adding a saturated aqueous ammonium chloride solution (100 mL). The temperature was raised to room temperature, and tetrahydrofuran was removed by vacuum concentration at 60°C. Dichloromethane (500 mL) and deionized water (300 mL) were added, and the layers were separated. The product was purified by silica gel column chromatography (200-300 mesh silica gel, ethyl acetate: n-hexane = 1: 30 as the eluent). After elution, a white solid compound **CPD001-6** (20.29 g, purity: 99.12%, yield: 64.55%) was obtained by vacuum concentration at 60°C, mass spectrometry: 495.14 (M+H).

### Synthesis of compound CPD001-7

**CPD001-6** (18.00 g, 36.33 mmol), acetic acid (180 mL), and 36%-38% concentrated hydrochloric acid (18 mL) were added to a 500 mL single-necked round-bottom flask. The mixture was heated to 90°C and stirred for 3 h. The consumption of the starting material **CPD001-6** was monitored by TLC (ethyl acetate: petroleum ether = 1:30 as the developing solvent). The system was cooled to 60°C, and methanol (360 mL) was added. The mixture was filtered by suction, and the filter cake was rinsed with 50 mL of methanol to obtain 14.35g of a white solid. The solid was recrystallized once from toluene and methanol, and then dried under vacuum at 80°C to obtain a white solid compound **CPD001-7** (15.40 g, purity: 99.88%, yield: 88.76%), mass spectrometry: 477.17 (M+H).

### Synthesis of compound CPD001

**CPD001-7** (13.00 g, 27.23 mmol), **CPD001-8** (10.33 g, 28.59 mmol), tri(dibenzylideneacetone)dipalladium (0.75 g, 0.82 mmol), sodium tert-butoxide (3.92 g, 40.84 mmol), and dry toluene (200 mL) were added to a 500 mL single-necked round-bottom flask. The flask was purged with nitrogen three times whiling stirring at room temperature. Then, a 50% solution of tri-tert-butylphosphine in xylene (0.66 g, 1.64 mmol) was added under nitrogen protection. The temperature was then raised to 110°C, and the reaction was carried out for 2 h. The reaction was monitored by TLC (ethyl acetate: hexane = 1:20 as the developing agent) until the starting material **CPD001-7** was consumed. After the reaction mixture was cooled to room temperature, toluene (400 mL) was added. The mixture was washed with deionized water (3×300 mL), and the layers were separated. The product was purified by silica gel column chromatography (200-300 mesh silica gel, ethyl acetate: n-hexane = 1:20 as the eluent). After elution, a white solid, **CPD001** (16.52 g, purity: 99.88 %, yield: 80.06%) was obtained by vacuum concentration at 60°C, mass spectrometry. After sublimation purification of 16.52 g of the crude **CPD001,** sublimation-purified **CPD001** (13.58 g, purity: 99.94%, yield: 82.20%) was obtained, mass spectrometry: 758.02 (M+H). ¹H NMR (400 MHz, CDCl₃) δ 7.72 (d, J=7.6Hz, 1H), 7.60 (d, J=8.3Hz, 1H), 7.56 (d, J=7.9Hz, 2H), 7.50 (d, J=7.3Hz, 1H), 7.35-7.26 (m, 6H), 7.24-7.15 (m, 7H), 7.03-6.97 (m, 4H), 6.88 (d, J=8.3Hz, 1H), 6.76 (s, 1H), 6.65 (d, J=7.6Hz, 1H), 6.60 (m, 4H), 2.93 (m, 1H), 2.45 (s, 3H), 1.78-1.70 (m, 4H), 1.52 (m, 4H), 1.00 (s, 6H).

### Synthesis of compound CPD010

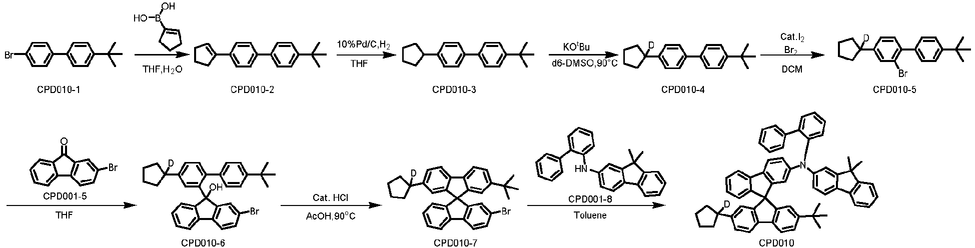

### Synthesis of compound CPD010-2

Referring to the synthesis and purification methods of compound **CPD001-2,** only the corresponding raw materials need to be changed to obtain the target compound **CPD010-2** (20.87 g, purity: 99.63%, yield: 93.33%), mass spectrometry: 277.18 (M+H).

### Synthesis of compound CPD010-3

Referring to the synthesis and purification methods of compound **CPD001-3,** only the corresponding raw materials need to be changed to obtain the target compound **CPD010-3** (19.09g, purity: 99.89%, yield: 96.87%), mass spectrometry: 279.22 (M+H).

### Synthesis of compound CPD010-4

**CPD010-3** (18.00 g, 64.65 mmol), deuterated dimethyl sulfoxide (180 mL), and potassium tert-butoxide (10.88 g, 96.97 mmol) were added to a 500 mL three-necked round-bottom flask. The flask was purged with nitrogen four times, and then heated to 90°C for reaction for 24 h. The reaction was monitored by nuclear magnetic resonance and mass spectrometry, conforming a deuterium substitution rate at the benzyl position to be greater than 99%.

After the reaction mixture was cooled to room temperature, deionized water (500 mL) was added, and a solid was precipitated. The solid was collected by suction filtration, and the filter cake was washed with deionized water (500 mL). After vacuum drying at 90°C, a white solid, **CPD010-4** (16.63 g, purity: 99.91%, deuterium substitution rate: 99.31%, yield: 92.05%) was obtained, mass spectrometry: 280.22 (M+H).

### Synthesis of compound CPD010-5

Referring to the synthesis and purification methods of compound **CPD001-4,** only the corresponding raw materials need to be changed to obtain the target compound **CPD010-5** (18.74 g, purity: 99.67%, yield: 90.09%), mass spectrometry: 358.14 (M+H).

### Synthesis of compound CPD010-6

Referring to the synthesis and purification methods of compound **CPD001-6,** only the corresponding raw materials need to be changed to obtain the target compound **CPD010-6** (17.50 g, purity: 99.00%, yield: 67.53%), mass spectrometry: 538.28 (M+H).

### Synthesis of compound CPD010-7

Referring to the synthesis and purification methods of compound **CPD001-7,** only the corresponding raw materials need to be changed to obtain the target compound **CPD010-7** (14.32 g, purity: 99.92%, yield: 89.88%), mass spectrometry: 520.27 (M+H).

### Synthesis of compound CPD010

Referring to the synthesis and purification methods of compound **CPD001,** only the corresponding raw materials need to be changed to obtain the target compound **CPD010** as a white solid (18.05 g, purity: 99.93%, yield: 81.11%). After sublimation purification of 18.05 g of the crude **CPD010,** sublimation-purified **CPD010** (13.68 g, purity: 99.95%, yield: 75.78%) was obtained, mass spectrometry: 801.52 (M+H). ¹H NMR (400 MHz, CDCl₃) δ 7.73 (d, J=7.46 Hz, 1H), 7.58-7.46 (m, 4H), 7.31-7.12 (m, 13H), 6.99-6.95 (m, 4H), 6.86-6.73 (m, 2H), 6.63-6.54 (m, 5H), 1.78-1.70 (m, 4H), 1.52 (m, 4H), 1.28 (s, 9H), 0.99 (s, 6H).

### Synthesis of compound CPD016

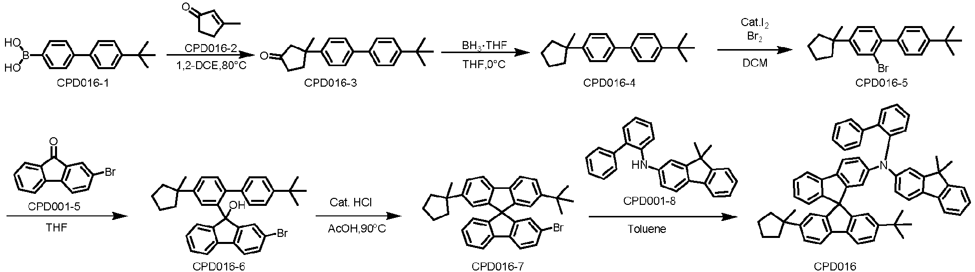

### Synthesis of compound CPD016-3

**CPD016-1** (20.00 g, 78.70 mmol), **CPD016-2** (11.35 g, 118.05 mmol), palladium trifluoroacetate (262.64 mg, 0.79 mmol), 2,2'-bipyridine (1.23 g, 7.87 mmol), and 1,2-dichloroethane (200 mL) were added into a 500 mL three-necked round-bottom flask. The flask was purged with nitrogen three times, and then heated to 80°C and for reaction for 24 h. The reaction of the starting materials was monitored by TLC (ethyl acetate: n-hexane = 1:10 as the developing agent). When the starting material **CPD016-1** was completely consumed, the heating was stopped. The reaction mixture was washed with deionized water (3×100 mL), and the layers were separated. The product was purified by silica gel column chromatography purification (200-300 mesh silica gel, ethyl acetate: n-hexane = 1:30 as the eluent). After elution, a white solid compound **CPD016-3** (18.72 g, purity: 99.59%, yield: 77.64%) was obtained by vacuum concentration at 60°C, mass spectrometry:307.20 (M+H).

### Synthesis of compound CPD016-4

**CPD016-3** (18.20 g, 59.39 mmol) and dried tetrahydrofuran solution (200 mL) were added to a 500-mL three-necked round-bottom flask. The flask was purged with nitrogen three times, and the system was then cooled to 0°C. A 1 mol/L solution of tetrahydrofuran borane complex (118.78 mL, 118.78 mmol) was added dropwise over 15 min. The reaction was maintained at 0°C for 1 h, monitored by TLC (ethyl acetate: n-heptane = 1:10 as the developing solvent) until the starting material **CPD016-3** was completely consumed. The reaction was quenched with 2 mol/L aqueous hydrochloric acid (100 mL), and tetrahydrofuran was removed by vacuum concentration at 60°C. Ethyl acetate (500 mL) was added, and the mixture was washed with deionized water (3×200 mL). The product was purified by silica gel column chromatography (200-300 mesh silica gel, ethyl acetate: n-heptane = 1:30 as the eluent). After elution, a white solid compound **CPD016-4** (14.77 g, purity: 99.93%, yield: 85.05%) was obtained by vacuum concentration at 60°C, mass spectrometry: 293.32 (M+H).

### Synthesis of compound CPD016-5

Referring to the synthesis and purification methods of **compound CPD001-4,** only the corresponding raw materials need to be changed to obtain the target compound **CPD016-5** (18.36 g, purity: 99.57%, yield: 89.73%), mass spectrometry: 371.23 (M+H).

### Synthesis of compound CPD016-6

Referring to the synthesis and purification methods of compound **CPD001-6,** only the corresponding raw materials need to be changed to obtain the target compound **CPD016-6** (18.06g, purity: 99.43%, yield: 62.17%), mass spectrometry: 551.21 (M+H).

### Synthesis of compound CPD016-7

Referring to the synthesis and purification methods of compound **CPD001-7,** only the corresponding raw materials need to be changed to obtain the target compound **CPD016-7** (14.86 g, purity: 99.91%, yield: 85.45%), mass spectrometry: 533.18 (M+H).

### Synthesis of compound CPD016

Referring to the synthesis and purification methods of compound **CPD001,** only the corresponding raw materials need to be changed to obtain the target compound **CPD016** (18.01 g, purity: 99.93%, yield: 78.32%). After sublimation purification of 18.01 g of the crude **CPD016,** sublimation-purified **CPD016** (purity: 99.96%, yield: 85.73%) was obtained, mass spectrometry: 814.04 (M+H). ¹H NMR (400 MHz, CDCl₃) δ7.71 (d, J=7.5Hz, 1H), 7.63 (d, J=8.3Hz, 1H), 7.55 (d, J=7.9Hz, 2H), 7.45 (d, J=7.1 Hz, 1H), 7.31-7.25 (m, 5H), 7.23-7.09 (m, 8H), 6.97-6.89 (m, 5H), 6.72 (s, 1H), 6.65 (s, 1H), 6.61-6.56 (m, 3H), 6.49 (d, J=8.1 Hz, 1H), 1.72-1.64 (m, 8H), 1.22 (s, 9H), 1.15 (s, 3H), 0.99 (s, 6H).

### Synthesis of compound CPD018

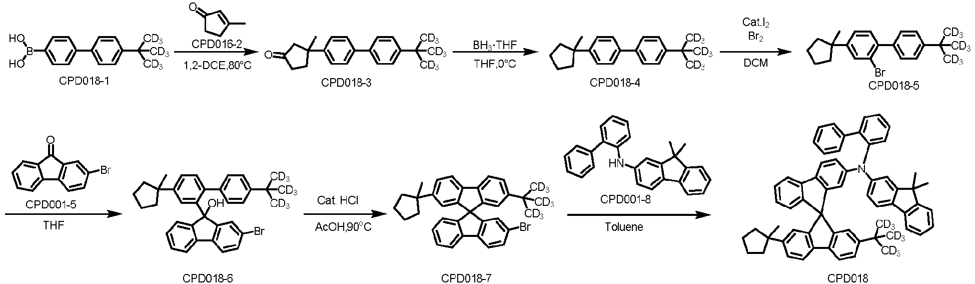

### Synthesis of compound CPD018-3

Referring to the synthesis and purification methods of compound **CPD016-3,** only the corresponding raw materials need to be changed to obtain the target compound **CPD018-3** (25.33 g, purity: 99.74%, yield: 75.41%), mass spectrometry: 316.23 (M+H).

### Synthesis of compound CPD018-4

Referring to the synthesis and purification methods of compound **CPD016-4,** only the corresponding raw materials need to be changed to obtain the target compound **CPD018-4** (22.15 g, purity: 99.91%, yield: 82.66%), mass spectrometry: 302.21 (M+H).

### Synthesis of compound CPD018-5

Referring to the synthesis and purification methods of compound **CPD001-4,** only the corresponding raw materials need to be changed to obtain the target compound **CPD018-5** (21.52 g, purity: 99.68%, yield: 81.01%), mass spectrometry: 380.20 (M+H).

### Synthesis of compound CPD018-6

Referring to the synthesis and purification methods of compound **CPD001-6,** only the corresponding raw materials need to be changed to obtain the target compound **CPD018-6** (20.17 g, purity: 99.60%, yield: 63.53%), mass spectrometry: 560.21 (M+H).

### Synthesis of compound CPD018-7

Referring to the synthesis and purification methods of compound **CPD001-7,** only the corresponding raw materials need to be changed to obtain the target compound **CPD018-7** (17.09 g, purity: 99.93%, yield: 88.74%), mass spectrometry: 542.42 (M+H).

### Synthesis of compound CPD018

Referring to the synthesis and purification methods of compound **CPD001,** only the corresponding raw materials need to be changed to obtain the target compound **CPD018** (19.28 g, purity: 99.93%, yield: 80.07%). After sublimation purification of 19.28 g of the crude **CPD018,** sublimation-purified **CPD018** (15.85 g, purity: 99.94%, yield: 82.20%) was obtained, mass spectrometry: 823.38 (M+H). ¹H NMR (400 MHz, CDCl₃) δ 7.73-7.48 (m, 6H), 7.31-7.20 (m, 11H), 7.01-6.95 (m, 7H), 6.73-6.58 (m, 5H), 1.77-1.71 (m, 4H), 1.62-1.54 (m, 4H), 1.28 (s, 3H), 1.00 (s, 6H).

### Synthesis of compound CPD028

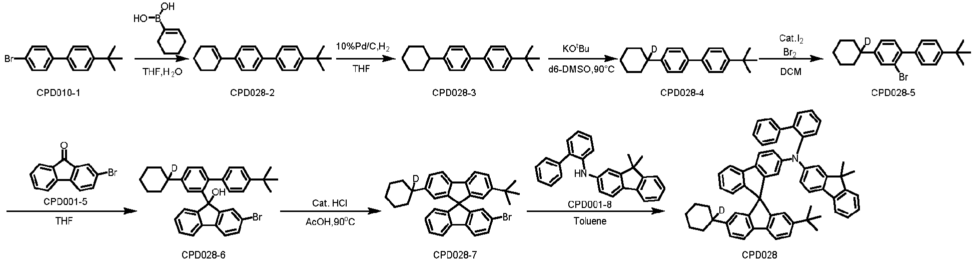

### Synthesis of compound CPD028-2

Referring to the synthesis and purification methods of **CPD001-2,** only the corresponding raw materials need to be changed to obtain the target compound **CPD028-2** (25.14 g, purity: 99.41%, yield: 91.11%), mass spectrum: 291.20 (M+H).

### Synthesis of compound CPD028-3

Referring to the synthesis and purification methods of compound **CPD001-3,** only the corresponding raw materials need to be changed to obtain the target compound **CPD028-3** (24.51 g, purity: 99.82%, yield: 94.23%), mass spectrometry: 293.22 (M+H).

### Synthesis of compound CPD028-4

Referring to the synthesis and purification methods of compound **CPD010-4,** only the corresponding raw materials need to be changed to obtain the target compound **CPD028-4** (20.91 g, purity: 99.92%, deuterium substitution rate: 99.45%, yield: 95.06%), mass spectrometry: 294.22 (M+H).

### Synthesis of compound CPD028-5

Referring to the synthesis and purification methods of compound **CPD001-4,** only the corresponding raw materials need to be changed to obtain the target compound **CPD028-5** (22.54 g, purity: 99.54%, yield: 88.04%), mass spectrometry: 372.44 (M+H).

### Synthesis of compound CPD028-6

Referring to the synthesis and purification methods of compound **CPD001-6,** only the corresponding raw materials need to be changed to obtain the target compound **CPD028-6** (19.87 g, purity: 99.30%, yield: 66.13%), mass spectrometry: 552.18 (M+H).

### Synthesis of compound CPD028-7

Referring to the synthesis and purification methods of compound **CPD001-7,** only the corresponding raw materials need to be changed to obtain the target compound **CPD028-7** (16.52 g, purity: 99.90%, yield: 85.51%), mass spectrometry: 534.18 (M+H).

### Synthesis of compound CPD028

Referring to the synthesis and purification methods of compound **CPD001,** only the corresponding raw materials need to be changed to obtain the target compound **CPD028** (19.57 g, purity: 99.93%, yield: 82.53%). After sublimation purification of 19.57 g of the crude **CPD028,** sublimation-purified **CPD028** (15.75 g, purity: 99.95%, yield: 80.48%) was obtained, mass spectrometry: 815.44 (M+H). ¹H NMR (400 MHz, CDCl₃) δ 7.72 (d, J=7.46 Hz, 1H), 7.63-7.56 (m, 6H), 7.48-7.32 (m, 10H), 7.02-6.95 (m, 4H), 6.86-6.54 (m, 8H), 1.78-1.69 (m, 4H), 1.53-1.43 (m, 6H), 1.28 (s, 9H), 1.02 (s, 6H).

### Synthesis of compound CPD049

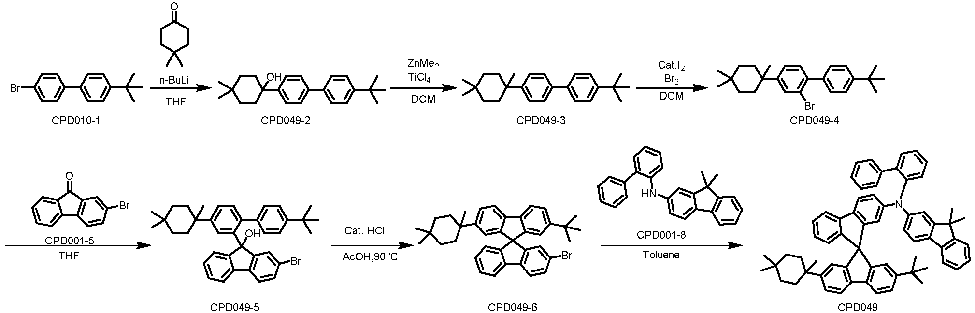

### Synthesis of compound CPD049-2

**CPD010-1** (30.00 g, 103.72 mmol) and dry tetrahydrofuran (300 mL) were added to a 1,000 mL three-necked round-bottom flask. The flask was purged with nitrogen three times, and was cooled with liquid nitrogen to -78°C. A 2.5mol/L solution of n-butyllithium in hexane (45.64 mL, 114.10 mmol) was added dropwise over 20 min, and the reaction was then maintained at -78°C for reaction for 1 h. 4,4-dimethylcyclohexanone (15.05 g, 119.29 mmol) was directly added dropwise over 3 min. The mixture was stirred at -78°C, and the reaction was monitored by TLC (hexane as the developing agent). After 1 h, the starting material **CPD010-1** was completely consumed, and most of the compound **CPD049-2** was generated.

A saturated aqueous ammonium chloride solution (200 mL) was added at -78°C to quench the reaction. The temperature was raised to room temperature, and tetrahydrofuran was removed by concentration. Dichloromethane (600 mL) was added, and the mixture was washed with deionized water (3×300 mL). The layers were separated, and the product was purified by silica gel column chromatography (200-300 mesh silica gel, ethyl acetate: n-hexane = 1:10 as the eluent). After vacuum concentration at 65°C, a colorless liquid compound **CPD049-2** (23.76 g, purity: 99.01%, yield: 68.08%) was obtained, mass spectrometry: 337.26 (M+H).

### Synthesis of compound CPD049-3

Titanium tetrachloride (33.82 g, 178.29 mmol) and dry dichloromethane (340 mL) were added into a dry 1,000 mL three-necked round-bottom flask. The flask was purged with nitrogen four times, and the system was then stirred and cooled to 0°C. Next, a 2 mol/L solution of dimethylzinc in toluene (89.15 mL, 178.29 mmol) was added dropwise over 15 min, and the reaction was maintained at 0°C for 30 min. **CPD049-2** (20.00 g, 59.43 mmol) was dissolved in dry dichloromethane (400 mL), and the solution was then added dropwise to the above system at 0°C for 30 min. The mixture was allowed to naturally warm to room temperature and stirred overnight for 12 h. The reaction was monitored by TLC (ethyl acetate: n-hexane = 1:10 as the developing agent) until the starting material **CPD049-2** was completely consumed. The system was reduced to 0°C, and deionized water (200 mL) was added to quench the reaction. The layers were separated, and the organic phase was washed with deionized water (3×200 mL). Silica gel column chromatography (200-300 mesh silica gel, n-hexane as the eluent) was performed. After elution, a white solid compound **CPD049-3** (14.97 g, purity: 99.86%, yield: 75.29%) was obtained by vacuum concentration at 65°C, mass spectrometry: 335.28 (M+H).

### Synthesis of compound CPD049-4

Referring to the synthesis and purification methods of compound **CPD001-4,** only the corresponding raw materials need to be changed to obtain the target compound **CPD049-4** (18.63 g, purity: 99.75%, yield: 89.66%), mass spectrometry: 413.26 (M+H).

### Synthesis of compound CPD049-5

Referring to the synthesis and purification methods of compound **CPD001-6,** only the corresponding raw materials need to be changed to obtain the target compound **CPD049-5** (17.49 g, purity: 99.33%, yield: 66.66%), mass spectrometry: 593.23 (M+H).

### Synthesis of compound CPD049-6

Referring to the synthesis and purification methods of compound **CPD001-7,** only the corresponding raw materials need to be changed to obtain the target compound **CPD049-6** (15.22 g, purity: 99.93%, yield: 87.07%), mass spectrometry: 575.22 (M+H).

### Synthesis of compound CPD049

Referring to the synthesis and purification methods of compound **CPD001,** only the corresponding raw materials need to be changed to obtain the target compound CPD049 as a white solid (15.60 g, purity: 99.93%, yield: 80.08%). After sublimation purification of 15.60 g of the crude **CPD049,** sublimation-purified **CPD049** (12.09 g, purity: 99.93%, yield: 77.50%) was obtained, mass spectrometry: 856.42 (M+H). ¹H NMR (400 MHz, CDCl₃) δ 7.73-7.49 (m, 5H), 7.37-7.17 (m, 12H), 6.99-6.95 (m, 6H), 6.73-6.54 (m, 6H), 1.78-1.69 (m, 4H), 1.53-1.43 (m, 4H), 1.28 (m, 12H), 1.01 (s, 6H), 0.87 (s, 6H).

### Synthesis of compound CPD056

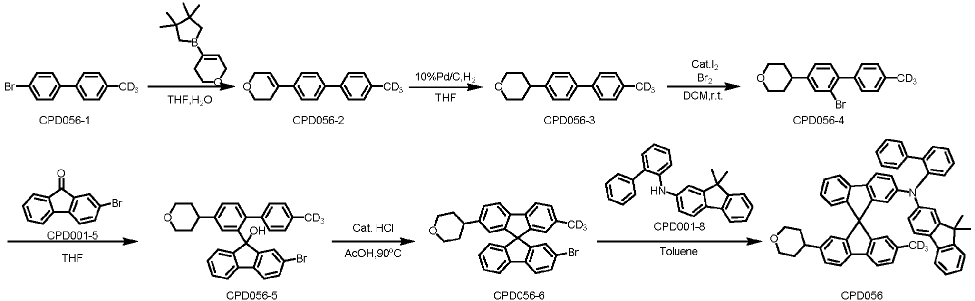

### Synthesis of compound CPD056-2

Referring to the synthesis and purification methods of compound **CPD001-2,** only the corresponding raw materials need to be changed to obtain the target compound **CPD056-2** (26.14 g, purity: 99.63%, yield: 93.15%), mass spectrometry: 254.16 (M+H).

### Synthesis of compound CPD056-3

Referring to the synthesis and purification methods of compound **CPD001-3,** only the corresponding raw materials need to be changed to obtain the target compound **CPD056-3** (22.01 g, purity: 99.89%, yield: 95.72%), mass spectrometry: 256.28 (M+H).

### Synthesis of compound CPD056-4

Referring to the synthesis and purification methods of compound **CPD001-4,** only the corresponding raw materials need to be changed to obtain the target compound **CPD056-4** (25.06 g, purity: 99.81%, yield: 88.07%), mass spectrometry: 334.08 (M+H).

### Synthesis of compound CPD056-5

Referring to the synthesis and purification methods of compound **CPD001-6,** only the corresponding raw materials need to be changed to obtain the target compound **CPD056-5** (24.89 g, purity: 99.00%, yield: 65.05%), mass spectrometry: 514.26 (M+H).

### Synthesis of compound CPD056-6

Referring to the synthesis and purification methods of compound **CPD001-7,** only the corresponding raw materials need to be changed to obtain the target compound **CPD056-6** (20.06 g, purity: 99.93%, yield: 85.63%), mass spectrometry: 496.13 (M+H).

### Synthesis of compound CPD056

Referring to the synthesis and purification methods of compound **CPD001,** only the corresponding raw materials need to be changed to obtain the target compound **CPD056** (21.08g, purity: 99.93%, yield: 82.51%). After sublimation purification of 21.08 g of the crude **CPD056,** sublimation-purified **CPD056** (16.46 g, purity: 99.94%, yield: 78.08%) was obtained, mass spectrometry: 777.36 (M+H). ¹H NMR (400 MHz, CDCl₃) δ 7.71 (d, J=7.62Hz, 1H), 7.57 (d, J=8.32Hz, 1H), 7.55 (m, 3H), 7.50-7.24 (m, 7H), 7.23-7.14 (m, 6H), 7.03-6.97 (m, 5H), 6.86-6.62 (m, 6H), 3.74 (m, 4H), 2.93-2.85 (m, 1H), 2.48-2.11 (m, 4H), 1.01 (s, 6H).

### Synthesis of compound CPD067

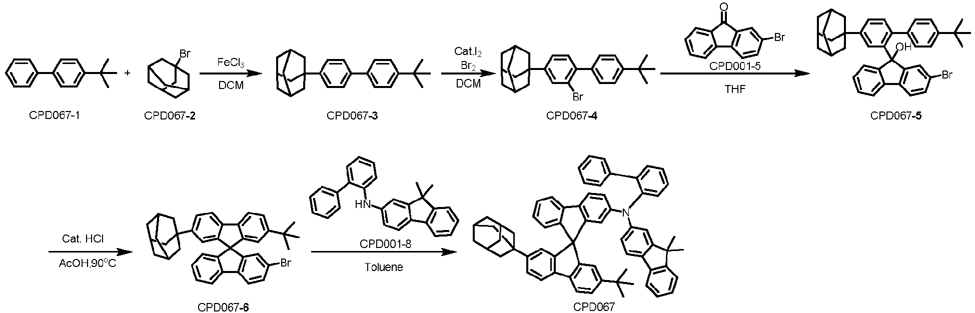

### Synthesis of compound CPD067-3

CPD067-1 (20.00 g, 95.09 mmol), anhydrous ferric chloride (1.54 g, 9.51 mmol), and dichloromethane (200 mL) were added to a 1,000 mL three-necked round-bottom flask, and stirred at room temperature. Then, 1-bromoadamantane (24.55 g, 114.11 mmol) was dissolved in dichloromethane (250 mL) and added dropwise to the above reaction system for 30 min, and the mixture was stirred at room temperature overnight for 12 h. The reaction was monitored by TLC (hexane as the developing agent) until the starting material **CPD067-1** was completely consumed. The reaction mixture was washed with deionized water (3 ×150 mL), and the layers were separated. The organic phase was concentrated and then purified by silica gel column chromatography (200-300 mesh silica gel, n-hexane as the eluent). After elution, a white solid **CPD067-3** (28.00 g, purity: 99.93%, yield: 85.45%) was obtained by vacuum concentration at 65°C, mass spectrum: 345.26 (M+H).

### Synthesis of compound CPD067-4

Referring to the synthesis and purification methods of compound **CPD001-4,** only the corresponding raw materials need to be changed to obtain the target compound **CPD067-4** (31.04 g, purity: 99.74%, yield: 89.56%, mass spectrometry: 423.18 (M+H).

### Synthesis of compound CPD067-5

Referring to the synthesis and purification methods of compound **CPD001-6,** only the corresponding raw materials need to be changed to obtain the target compound **CPD067-5** (30.51 g, purity: 99.36%, yield: 66.10%), mass spectrometry: 603.22 (M+H).

### Synthesis of compound CPD067-6

Referring to the synthesis and purification methods of compound **CPD001-7,** only the corresponding raw materials need to be changed to obtain the target compound **CPD067-6** (27.87 g, purity: 99.72%, yield: 87.71%), mass spectrometry: 585.62 (M+H).

### Synthesis of compound CPD067

Referring to the synthesis and purification methods of compound **CPD001,** only the corresponding raw materials need to be changed to obtain the target compound **CPD067** (29.22 g, purity: 99.91%, yield: 81.23%). After sublimation purification of 29.22 g of the crude **CPD067,** sublimation-purified **CPD067** (20.45 g, purity: 99.93%, yield: 69.98%) was obtained, mass spectrometry: 866.44 (M+H). ¹H NMR (400 MHz, CDCl₃) δ 7.72 (d, J=7.64 Hz, 1H), 7.57-7.55 (m, 4H), 7.50-7.24 (m, 7H), 7.23-7.14 (m, 6H), 7.03-6.92 (m, 5H), 6.86-6.68 (m, 6H), 1.81-1.78 (m, 5H), 1.51-1.48 (m, 10H), 1.27 (s, 9H), 1.00 (s, 6H).

### Synthesis of compound CPD073

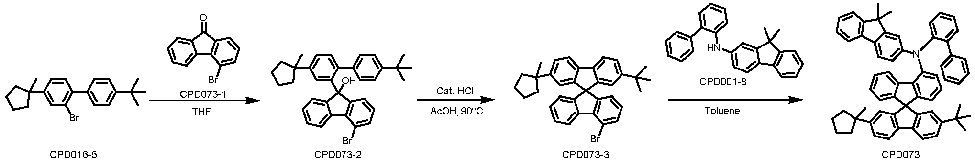

### Synthesis of compound CPD073-2

Referring to the synthesis and purification methods of compound **CPD001-6,** only the corresponding raw materials need to be changed to obtain the target compound **CPD073-2** (23.30 g, purity: 99.67%, yield: 65.29%), mass spectrometry: 551.20 (M+H).

### Synthesis of compound CPD073-3

Referring to the synthesis and purification methods of compound **CPD001-7,** only the corresponding raw materials need to be changed to obtain the target compound **CPD073-3** (21.01 g, purity: 99.93%, yield: 87.70%), mass spectrometry: 533.18 (M+H).

### Synthesis of compound CPD073

Referring to the synthesis and purification methods of compound **CPD001,** only the corresponding raw materials need to be changed to obtain the target compound CPD073 (19.87 g, purity: 99.92%, yield: 82.15%). After sublimation purification of 19.87 g of the crude **CPD073,** sublimation-purified **CPD073** (15.01 g, purity: 99.95%, yield: 75.54%) was obtained, mass spectrometry: 814.23 (M+H). ¹H NMR (400 MHz, CDCl₃) δ 7.71-7.68 (m, 2H), 7.52-7.51 (m, 2H), 7.49-7.48 (m, 2H), 7.24-7.13 (m, 4H), 7.06-6.94 (m, 9H), 6.91-6.80 (m, 6H), 6.77-6.60 (m, 4H), 1.78-1.70 (m, 4H), 1.52 (m, 4H), 1.28-1.26 (m, 12H), 1.01 (s, 6H).

### Synthesis of compound CPD088

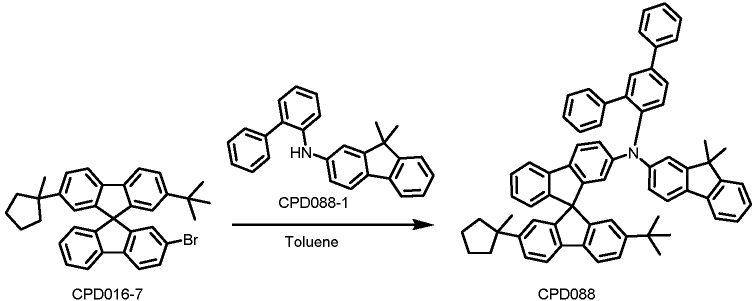

### Synthesis of compound CPD088

Referring to the synthesis and purification methods of compound **CPD001,** only the corresponding raw materials need to be changed to obtain the target compound **CPD088** as a white solid (23.03 g, purity: 99.92%, yield: 80.06%). After sublimation purification of 23.03 g of the crude **CPD088,** sublimation-purified **CPD088** (18.86 g, purity: 99.92%, yield: 81.89%) was obtained, mass spectrometry: 890.16 (M+H). ¹H NMR (400 MHz, CDCl₃) δ 7.72-7.46 (m, 5H), 7.31-7.12 (m, 14H), 6.99-6.95 (m, 6H), 6.73-6.54 (m, 8H), 1.77-1.71 (m, 4H), 1.53 (m, 4H), 1.28 (m, 12H), 1.01 (s, 6H).

### Synthesis of compound CPD095

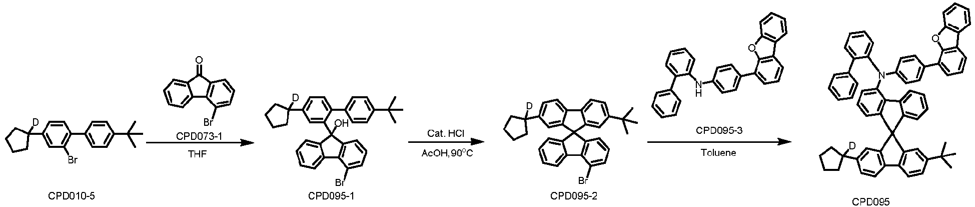

### Synthesis of compound CPD095-1

Referring to the synthesis and purification methods of compound **CPD073-2,** only the corresponding raw materials need to be changed to obtain the target compound **CPD095-1** (20.03 g, purity: 99.55%, yield: 65.02%), mass spectrometry: 538.12 (M+H).

### Synthesis of compound CPD095-2

Referring to the synthesis and purification methods of compound **CPD073-3,** only the corresponding raw materials need to be changed to obtain the target compound **CPD095-2** (19.86 g, purity: 99.91%, yield: 85.23%), mass spectrometry: 520.20 (M+H).

### Synthesis of compound CPD095

Referring to the synthesis and purification methods of compound **CPD001,** only the corresponding raw materials need to be changed in order to obtain the target compound **CPD095** (18.75 g, purity: 99.92%, yield: 85.77%). After sublimation purification of 18.75 g of the crude **CPD095,** sublimation-purified **CPD095** (14.24 g, purity: 99.94%, yield: 76.14%) was obtained, mass spectrometry: 851.42 (M+H). ¹H NMR (400 MHz, CDCl₃) δ 8.02 (d, J=7.86 Hz, 2H), 7.86-7.62 (m, 8H), 7.37-7.22 (m, 6H), 7.20-7.12 (m, 6H), 6.99-6.95 (m, 4H), 6.86-6.73 (m, 2H), 6.63-6.54 (m, 5H), 1.78-1.70 (m, 4H), 1.52 (m, 4H), 1.28 (s, 9H).

### Synthesis of compound CPD105

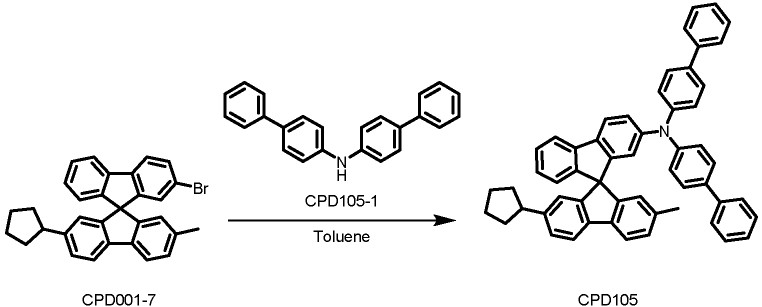

### Synthesis of compound CPD105

Referring to the synthesis and purification methods of compound **CPD001,** only the corresponding raw materials need to be changed to obtain the target compound **CPD105** as a white solid (17.57 g, purity: 99.93%, yield: 86.12%). After sublimation purification of 17.57 g of the crude **CPD105,** sublimation-purified **CPD105** (12.30 g, purity: 99.95%, yield: 70.00%) was obtained, mass spectrometry: 718.14 (M+H). ¹H NMR (400 MHz, CDCl₃) δ 7.72-7.68 (m, 2H), 7.60-7.56 (m, 8H), 7.48-7.35 (m, 4H), 7.30-7.20 (m, 9H), 7.03-6.97 (m, 4H), 6.88-6.65 (m, 4H), 2.93 (m, 1H), 2.45 (s, 3H), 1.78-1.70 (m, 4H), 1.52-1.49 (m, 4H).

### Synthesis of compound CPD113

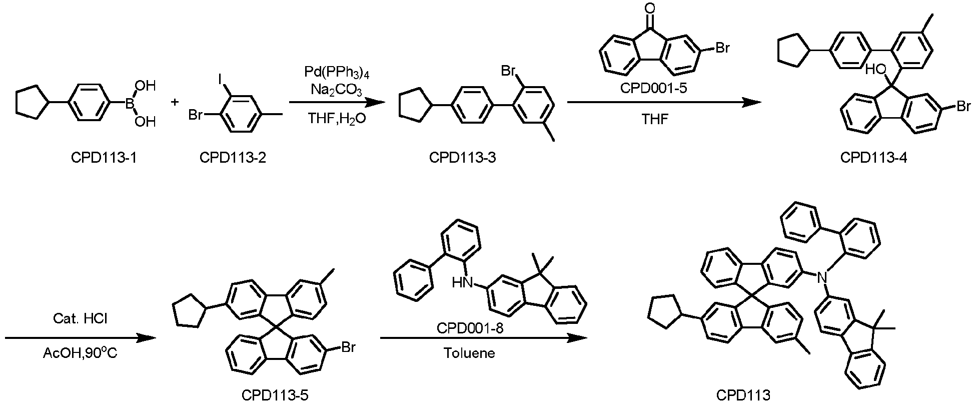

### Synthesis of compound CPD113-3

**CPD113-1** (20.00 g, 105.24 mmol), **CPD113-2** (37.50 g, 126.28 mmol), tetrakis(triphenylphosphine)palladium (2.43g, 2.10 mmol), sodium carbonate (16.73g, 157.86 mmol), tetrahydrofuran (200 mL), and deionized water (80 mL) were added into a 500 mL three-necked round-bottom flask. The flask was purged with nitrogen three times, and heated to 66°C for reaction for 8 h. The consumption of the starting material **CPD 113-1** was monitored by TLC (n-hexane as the expanding agent). After the reaction, the mixture was cooled to room temperature. Tetrahydrofuran was removed by concentration at 60°C, Ethyl acetate (500 mL) was added, and the mixture was washed with deionized water (3×150ml). The layers were separated, and the organic phase was concentrated. Silica gel column chromatography (200-300 mesh silica gel, n-hexane as the eluent) was performed. After elution, a white solid compound **CPD113-3** (28.40 g, purity: 99.90%, yield: 85.61%) was obtained by vacuum concentration at 65°C, mass spectrometry: 315.06 (M+H).

### Synthesis of compound CPD113-4

Referring to the synthesis and purification methods of compound **CPD001-6,** only the corresponding raw materials need to be changed to obtain the target compound **CPD113-4** (25.62 g, purity: 99.01%, yield: 65.91%), mass spectrometry: 495.10 (M+H).

### Synthesis of compound CPD113-5

Referring to the synthesis and purification methods of compound **CPD001-7,** only the corresponding raw materials need to be changed in order to obtain the target compound **CPD113-5** (24.33 g, purity: 99.91%, yield: 83.16%), mass spectrometry: 477.21 (M+H).

### Synthesis of compound CPD113

Referring to the synthesis and purification methods of compound **CPD001,** only the corresponding raw materials need to be changed in order to obtain the target compound **CPD113** (27.88 g, purity: 99.95%, yield: 82.11%). After sublimation purification of 27.88 g of the crude **CPD113,** sublimation-purified **CPD113** (20.94 g, purity: 99.95%, yield: 75.10%) was obtained, mass spectrometry: 758.16 (M+H). ¹H NMR (400 MHz, CDCl₃) δ 7.73 (d, J=7.63 Hz, 1H), 7.61-7.52 (m, 4H), 7.35 (s, 1H), 7.24-7.15 (m, 12H), 7.03-6.97 (m, 5H), 6.88-6.60 (m, 6H), 2.93 (m, 1H), 2.44 (s, 3H), 1.78-1.50 (m, 8H), 0.99 (s, 6H).

### Synthesis of compound CPD123

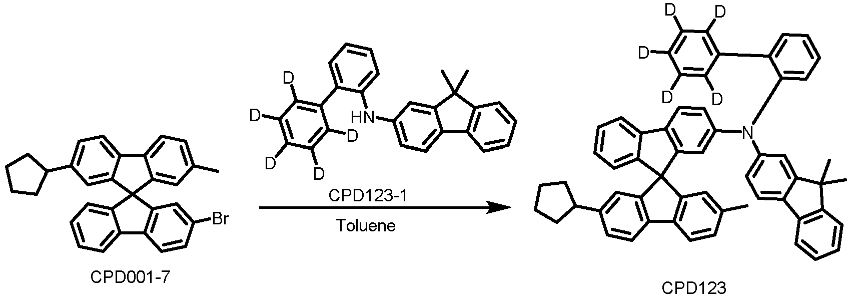

### Synthesis of compound CPD123

Referring to the synthesis and purification methods of compound **CPD001,** only the corresponding raw materials need to be changed to obtain the target compound **CPD123** (12.35 g, purity: 99.92%, yield: 85.11%). After sublimation purification of 12.35 g of the crude **CPD123,** sublimation-purified **CPD 123** (9.36 g, purity: 99.92%, yield: 75.78%) was obtained, mass spectrometry: 763.40(M+H). ¹H NMR (400 MHz, CDCl₃) δ 7.71 (d, J=7.7 Hz, 1H), 7.58-7.43 (m, 6H), 7.29-7.02 (m, 13H), 6.88-6.60 (m, 4H), 2.86 (m, 1H), 2.42 (s, 3H), 1.76-1.50 (m, 8H), 0.98 (s, 6H).

### Application Example: Production of Organic Electroluminescent Devices

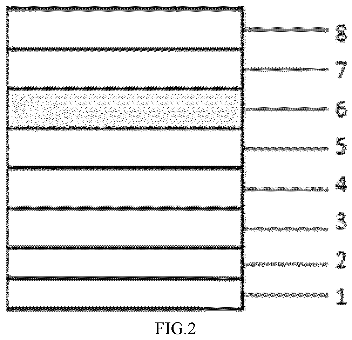

As shown in Fig. 2, the organic electroluminescent device consists of a stacked structure including a glass substrate 1, an anode 2, a hole injection layer 3, a first hole transport layer 4 (HTL1), a second hole transport layer 5 (HTL2), a light-emitting layer 6, an electron transport layer 7 (ETL), and a cathode 8.

The glass substrate (50 mm × 50 mm × 1.0 mm) with an ITO (100 nm) transparent electrode (anode 2) was ultrasonically cleaned in ethanol for 10 min, dried at 150°C, and finally treated with N₂ Plasma for 30 min. The cleaned glass substrate was installed on the substrate holder of a vacuum deposition device. Firstly, a compound HATCN (hole injection layer 3) was deposited on the side with transparent electrode lines in a manner that covered the transparent electrode, forming a thin film with a thickness of 5 nm. Next, a layer of HTM1 was deposited to form a thin film with a thickness of 60 nm as HTL1 (first hole transport layer 4). Then, a layer of HTM2 was deposited on the HTM1 thin film to form a thin film with a thickness of 10 nm as HTL2 (second hole transport layer 5). Then, a host material and a dopant material were co-deposited on the HTM2 film layer in a ratio of 98%:2%, forming a film with a thickness of 25 nm (emission layer 6). On the light-emitting layer, ETL: EIL (5:5, 30 nm) was sequentially deposited according to the following table as the electron transport material (electron transport layer 7). Then, Mg/Ag (100 nm, 1:9) was co-deposited as the cathode material (cathode 8).

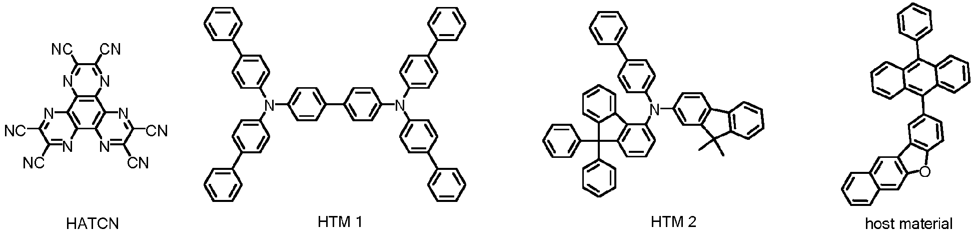

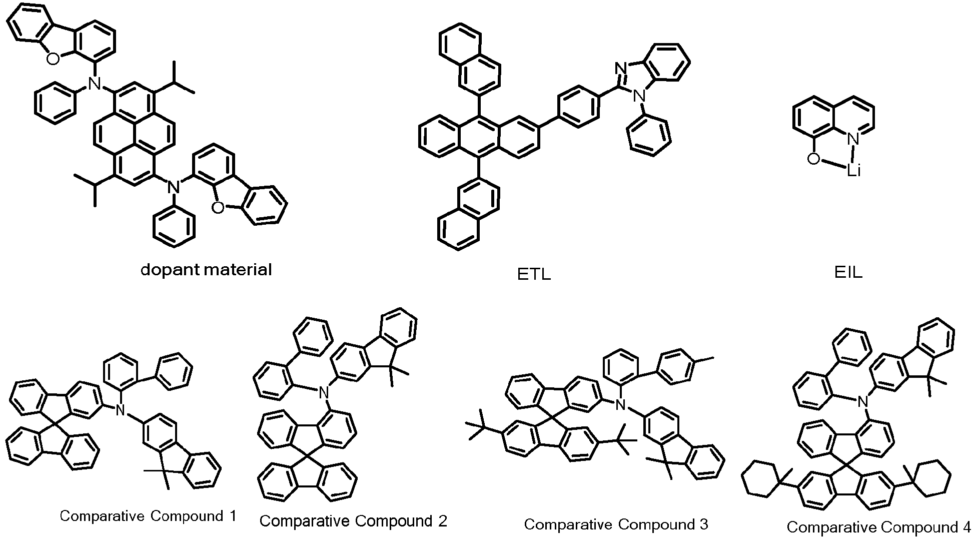

### Evaluation

The device performance testing was conducted on the above devices. The compounds of the examples in the present disclosure and comparative examples 1-4 were respectively used as the HTL layers for comparison. A constant current power supply (Keithley 2400) was used to pass a fixed current density through the light-emitting element, and a spectroradiometer (CS 2000) was used to test the emission spectrum. Meanwhile, at a current density of 20 mA/cm², the device voltage, current efficiency, and the time for the brightness to decay to 90% of the initial value (LT90) were measured. The results were shown in the table below.

| | HTL1 | HTL2 | Starting voltage (v) | Current efficiency (Cd/a) | LT90 |
|---|---|---|---|---|---|
| | | | | | @1000nits |
| Example 1 | CPD001 | HTM2 | 3.76 | 8.52 | 146 |
| Example 2 | CPD010 | HTM2 | 3.68 | 8.64 | 167 |
| Example 3 | CPD016 | HTM2 | 3.66 | 8.67 | 154 |
| Example 4 | CPD018 | HTM2 | 3.73 | 8.55 | 178 |
| Example 5 | CPD028 | HTM2 | 3.65 | 8.58 | 135 |
| Example 6 | CPD049 | HTM2 | 3.69 | 8.76 | 153 |
| Example 7 | CPD056 | HTM2 | 3.58 | 8.61 | 143 |
| Example 8 | CPD067 | HTM2 | 3.80 | 8.58 | 156 |
| Example 9 | CPD073 | HTM2 | 3.78 | 8.57 | 136 |
| Example 10 | CPD088 | HTM2 | 3.81 | 8.59 | 188 |
| Example 11 | CPD095 | HTM2 | 3.82 | 8.55 | 148 |
| Example 12 | CPD0105 | HTM2 | 3.79 | 8.63 | 152 |
| Example 13 | CPD113 | HTM2 | 3.74 | 8.58 | 135 |
| Example 14 | CPD123 | HTM2 | 3.72 | 8.64 | 166 |
| Example 15 | HTM1 | CPD073 | 3.75 | 8.63 | 128 |
| Example 16 | CPD016 | CPD073 | 3.68 | 8.81 | 144 |
| Comparative example 1 | HTM1 | HTM2 | 3.92 | 8.34 | 72 |
| Comparative example 2 | Comparative Compound 1 | HTM2 | 3.89 | 8.48 | 90 |
| Comparative example 3 | Comparative Compound 2 | HTM2 | 4.11 | 8.24 | 71 |
| Comparative example 4 | Comparative Compound 3 | HTM2 | 3.96 | 8.33 | 103 |
| Comparative example 5 | Comparative Compound 4 | HTM2 | 3.88 | 8.32 | 86 |
| Comparative example 6 | HTM1 | Comparative Compound 4 | 4.01 | 8.34 | 103 |

### Comparison of sublimation temperature

The sublimation temperature was defined as the temperature corresponding to a deposition rate of 1 angstrom per second at a vacuum level of 10⁻⁷ Torr. The test results were as follows:

| Material | Sublimation temperature/°C |
|---|---|
| CPD001 | 262 |
| CPD016 | 265 |
| CPD056 | 266 |
| CPD073 | 268 |
| Comparative compound 1 | 268 |
| Comparative compound 2 | 268 |
| Comparative compound 3 | 272 |
| Comparative compound 4 | 281 |
| HTM1 | 380 |
| HTM2 | 275 |

Form the data comparison in the table above, it can be seen that the hole transport material in the present disclosure has a lower sublimation temperature, which is beneficial for industrial application.

### Comparison of Lateral Carrier Mobility

A 50 mm × 50 mm × 1.0 mm glass substrate was modified to have ITO (100 nm) transparent electrodes at both ends and Mg/Ag (100 nm, 1:9) cathode materials, with a 5 mm × 5 mm × 0.4 mm groove in the middle. The substrate was ultrasonically cleaned in ethanol for 10 minutes, dried at 150 °C, and then treated with N₂ plasma for 30 minutes. The cleaned glass substrate was mounted on the substrate holder of a vacuum evaporation device. First, an HTL1 layer with a thickness of 10 nm was evaporated on the side with the transparent electrodes in a way that covered the transparent electrodes (3% HATCN was doped into CPD016, CPD056, Comparative Compounds 1-4, and HTM1 respectively). Then, an HTL2 layer with a thickness of 100 nm was evaporated (CPD016, CPD056, Comparative Compounds 1-4, and HTM1 respectively). After encapsulation, the voltage-current curves were tested to obtain the lateral transmission current data. The lateral resistance value ratios of each material were calculated (calculated with Comparative Compound 1 as 100%).

It can be observed that when the test voltage is 15 V, the lateral resistance values of CPD016 and CPD056 are relatively high, both higher than those of Comparative Compounds 1-4 and HTM1. It indicates that the carrier lateral mobility is low, and the lateral crosstalk current is small, which is beneficial for better low-gray-scale color purity.

| HTL1 | HTL2 | Lateral Resistance Ratio (%) |
|---|---|---|
| 3% HATCN: 97% CPD016 | CPD016 | 153% |
| 3% HATCN: 97% CPD056 | CPD056 | 168% |
| 3% HATCN: 97% Comparative Compound 1 | Comparative Compound 1 | 100% |
| 3% HATCN: 97% Comparative Compound 2 | Comparative Compound 2 | 93% |
| 3% HATCN: 97% Comparative Compound 3 | Comparative Compound 3 | 95% |
| 3% HATCN: 97% Comparative Compound 4 | Comparative Compound 4 | 110% |
| 3% HATCN: 97% HTM1 | HTM1 | 70% |

The materials in the present disclosure have the advantages such as high photoelectric stability, low sublimation temperature, low driving voltage, low lateral carrier mobility, high luminous efficiency, and long device service life, making it suitable for use in organic electroluminescent devices. The materials, particularly as hole injection and transport materials, has potential applications in the AMOLED industry.

## Claims

1. A spiro compound comprising an asymmetric alkyl substitution, having a structure of formula (1), 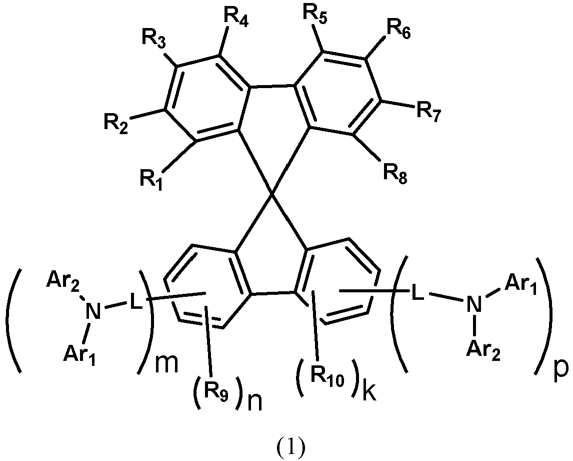
wherein R₁-R₁₀ are each independently selected from a group consisting of hydrogen, deuterium, halogen, cyano, hydroxy, thiol, amino, a substituted or unsubstituted C1-C10 alkyl, a substituted or unsubstituted C1-C10 heteroalkyl, a substituted or unsubstituted C3-C20 cycloalkyl, a substituted or unsubstituted C3-C20 heterocycloalkyl, a substituted or unsubstituted C2-C10 alkenyl, a substituted or unsubstituted C2-C10 alkynyl, a substituted or unsubstituted C6-C30 aryl, a substituted or unsubstituted C2-C30 heteroaryl, a substituted or unsubstituted (tri-C1-C10 alkyl)silyl, a substituted or unsubstituted (tri-C6-C12 aryl)silyl, a substituted or unsubstituted (di-C1-C10 alkyl)(mono-C6-C30 aryl)silyl, or a substituted or unsubstituted (mono-C1-C10 alkyl)(di-C6-C30 aryl)silyl; alternatively, two adjacent groups among R₁-R₈ and R₉-R₁₀ are connected to each other to form an aliphatic or aromatic ring structure;
wherein at least one of R₅-R₈ is a substituted or unsubstituted C1-C10 alkyl, or a substituted or unsubstituted C1-C10 heteroalkyl; and at least one of R₁-R₄ is a substituted or unsubstituted C3-C20 cycloalkyl, or a substituted or unsubstituted C3-C20 heterocycloalkyl;
wherein L is independently selected from a group consisting of a single bond, a substituted or unsubstituted C6-C30 arylene, or a substituted or unsubstituted C2-C30 heteroarylene;
wherein Ar1 and Ar2 are each independently selected from a substituted or unsubstituted C6-C30 aryl, or a substituted or unsubstituted C2-C30 heteroaryl;
wherein m, n, k and p are each independently selected from 0 or an integer from 1 to 4, with the proviso that m + n = 4 and p + k = 4;
wherein the heteroalkyl, the heterocycloalkyl, the heteroarylene, and the heteroaryl each comprises at least one heteroatom selected from O, N, Si, or S; and
wherein the substitution refers to being substituted with deuterium, halogen, C1-C6 alkyl, C3-C6 cycloalkyl, C1-C6 alkyl-substituted amino, cyano, isocyano or phosphino, with the number of substitutions being from a monosubstitution to a maximum substitution.

2. The spiro compound according to claim 1, wherein m + p = 1.

3. The spiro compound according to claim 2, having a structure represented by one of formulas (2) to (12), 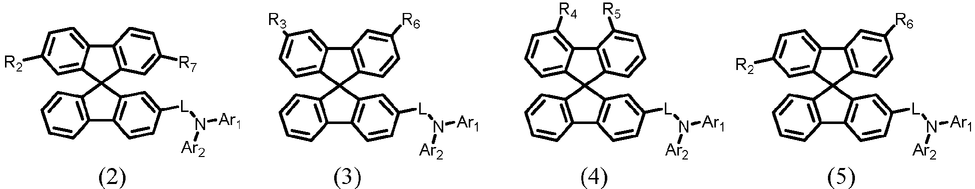 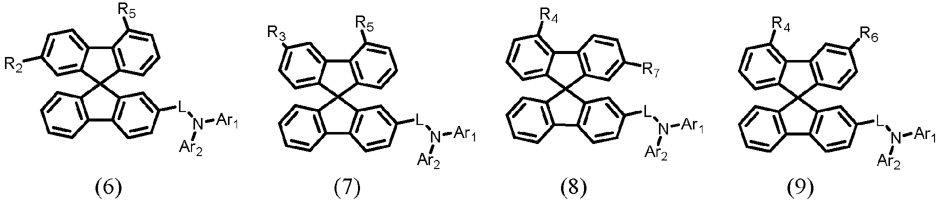 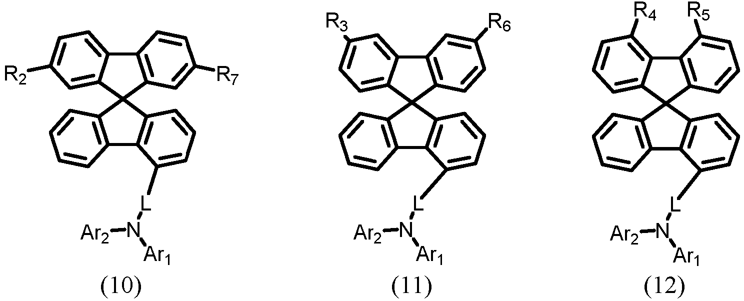 wherein each of R₅, R₆ and R₇ is a substituted or unsubstituted C1-C10 alkyl, or a substituted or unsubstituted C1-C10 heteroalkyl; and each of R₂, R₃ and R₄ is a substituted or unsubstituted C3-C20 cycloalkyl, or a substituted or unsubstituted C3-C20 heterocycloalkyl; and L, Ar1 and Ar2 are as defined in claim 1.

4. The spiro compound according to claim 3, wherein the L is a single bond.

5. The spiro compound according to claim 4, having a structure of formula (2) or formula (10).

6. The spiro compound according to claim 5, having a structure represented by one of formulas (13) to (14), 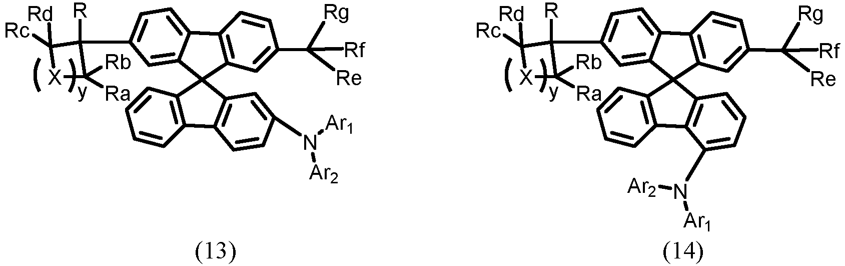
wherein X is independently selected from a group consisting of C(R₀)₂, Si(R₀)₂, O, S, or NR₀;
wherein y is independently 0 or an integer from 1 to 7; a three-membered ring is formed when y=0; each X is same or different when y≥2;
wherein R, R₀, and Ra-Rd are each independently selected from a group consisting of hydrogen, deuterium, halogen, cyano, isocyano, phosphino, amino, a substituted or unsubstituted C1-C10 alkyl, a substituted or unsubstituted C1-C10 heteroalkyl, or a substituted or unsubstituted C3-C20 cycloalkyl; alternatively, any two of Ra, Rb, Rc, Rd, and/or any two R₀ groups, and/or R and one of Ra, Rb, Rc, Rd, R₀, are interconnected to form a ring structure; and Re-Rg are each independently selected from a group consisting of hydrogen, deuterium, halogen, C1-C6 alkyl, C1-C6 alkyl-substituted amino, cyano, isocyano, or phosphino;
wherein the substitution refers to being substituted with deuterium, F, Cl, Br, C1-C6 alkyl, C3-C6 cycloalkyl, C1-C6 alkyl-substituted amino, cyano, isocyano, or phosphino, with the number of substitutions being from a monosubstitution to a maximum substitution.

7. The spiro compound according to claim 6, wherein R is hydrogen, deuterium, a substituted or unsubstituted C1-C10 alkyl, or a substituted or unsubstituted C1-C10 heteroalkyl.

8. The spiro compound according to claim 6, wherein y is a number greater than or equal to 2.

9. The spiro compound according to claim 8, wherein at most one X group in two or more X groups is selected from O, S, or NR₀.

10. The spiro compound according to claim 6, wherein two R₀ groups and/or R and R₀ are interconnected to form a ring structure.

11. The spiro compound according to any one of claims 1-10, wherein Ar1 and Ar2 are different, and Ar1 and Ar2 are each independently selected from a substituted or unsubstituted phenyl, a substituted or unsubstituted biphenyl, a substituted or unsubstituted naphthyl, a substituted or unsubstituted fluorenyl, a substituted or unsubstituted dibenzofuranyl, or a substituted or unsubstituted carbazolyl; wherein the substitution on the phenyl, biphenyl, naphthyl, fluorenyl, dibenzofuranyl, and carbazolyl refers to being substituted with deuterium, F, Cl, Br, C6-C10 aryl, C5-C12 heteroaryl, C1-C6 alkyl, or C3-C6 cycloalkyl; and the heteroaryl comprises at least one heteroatom selected from O, N, or S.

12. The spiro compound according to claim 11, being represented by one of the following structural formulas, or being correspondingly partially or completely deuterated or fluorinated:
| | | | |
|---|---|---|---|
| 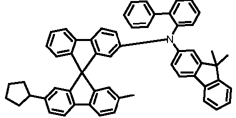 | 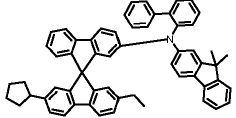 | 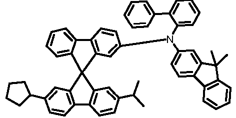 | 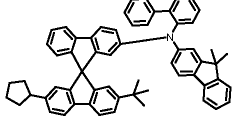 |
| CPD001 | CPD002 | CPD003 | CPD004 |
| 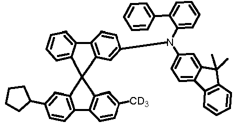 | 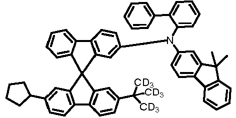 | 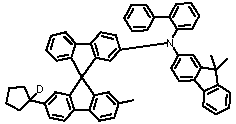 | 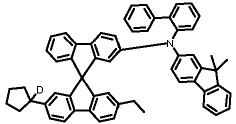 |
| CPD005 | CPD006 | CPD007 | CPD008 |
| 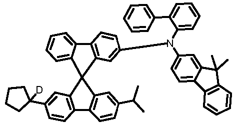 | 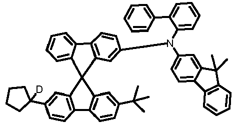 | 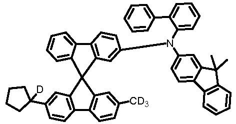 | 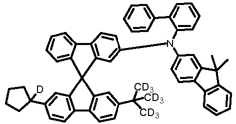 |
| CPD009 | CPD010 | CPD011 | CPD012 |
| 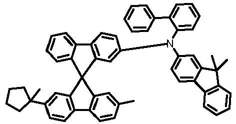 | 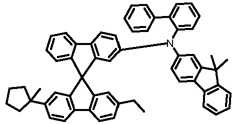 | 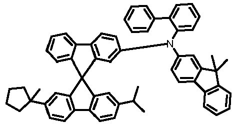 | 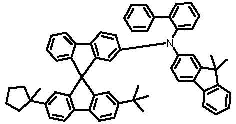 |
| CPD013 | CPD014 | CPD015 | CPD016 |
| 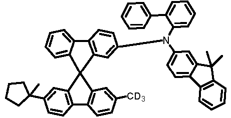 | 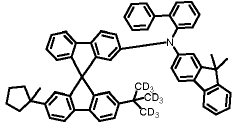 | 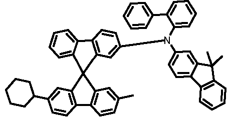 | 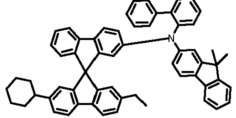 |
| CPD017 | CPD018 | CPD019 | CPD020 |
| 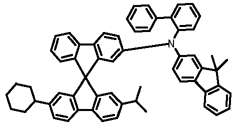 | 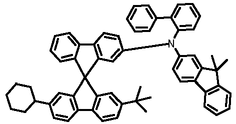 | 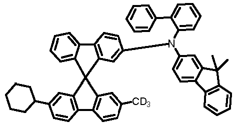 | 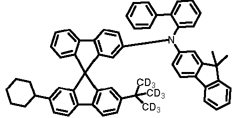 |
| CPD021 | CPD022 | CPD023 | CPD024 |
| 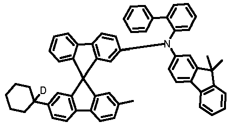 | 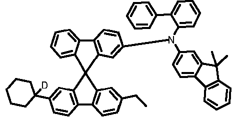 | 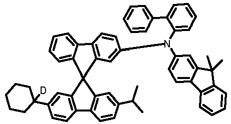 | 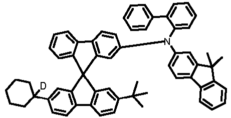 |
| CPD025 | CPD026 | CPD027 | CPD028 |
| 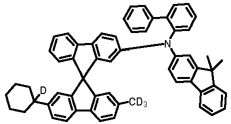 | 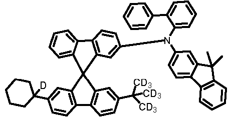 | 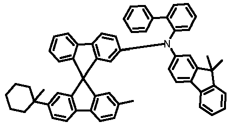 | 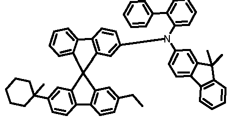 |
| CPD029 | CPD030 | CPD031 | CPD032 |
| 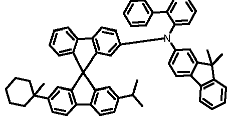 | 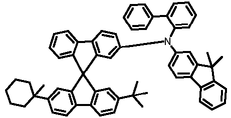 | 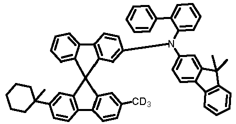 | 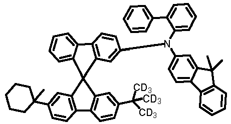 |
| CPD033 | CPD034 | CPD035 | CPD036 |
| 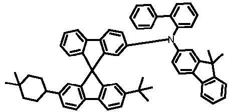 | 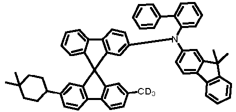 | 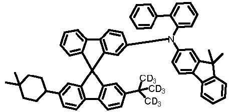 | 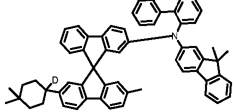 |
| CPD037 | CPD038 | CPD039 | CPD040 |
| 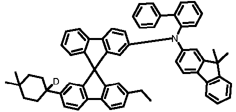 | 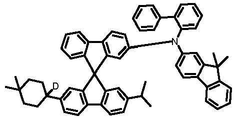 | 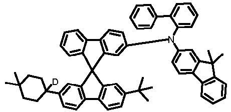 | 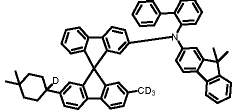 |
| CPD041 | CPD042 | CPD043 | CPD044 |
| 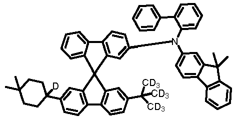 | 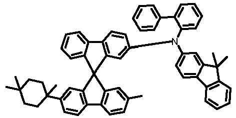 | 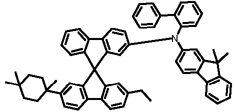 | 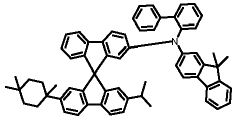 |
| CPD045 | CPD046 | CPD047 | CPD048 |
| 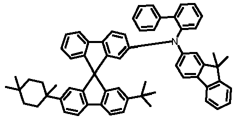 | 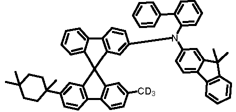 | 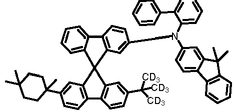 | 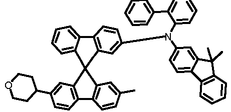 |
| CPD049 | CPD050 | CPD051 | CPD052 |
| 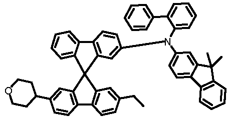 | 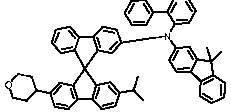 | 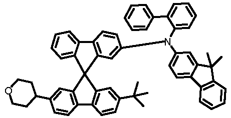 | 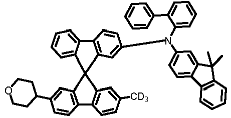 |
| CPD053 | CPD054 | CPD055 | CPD056 |
| 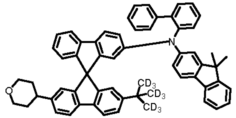 | 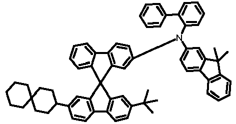 | 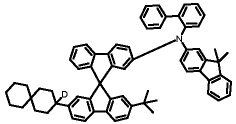 | 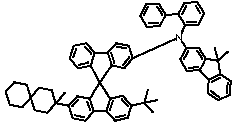 |
| CPD057 | CPD058 | CPD059 | CPD060 |
| 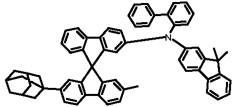 | 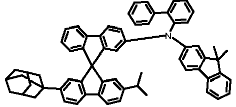 | 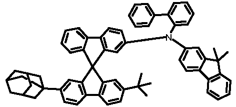 | 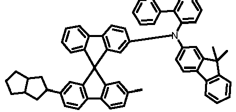 |
| CPD061 | CPD062 | CPD063 | CPD064 |
| 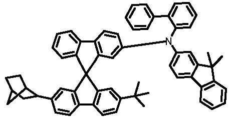 | 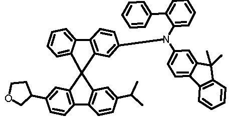 | 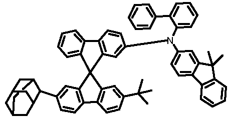 | 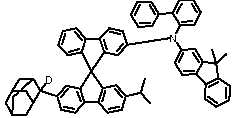 |
| CPD065 | CPD066 | CPD067 | CPD068 |
| 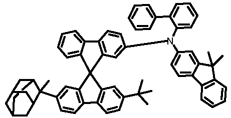 | 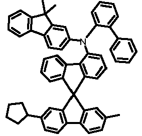 | 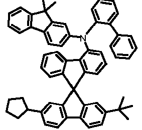 | 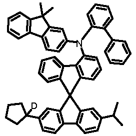 |
| CPD069 | CPD070 | CPD071 | CPD072 |
| 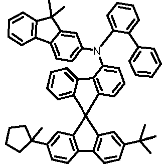 | 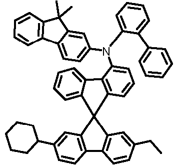 | 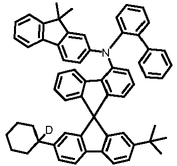 | 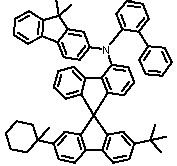 |
| CPD073 | CPD074 | CPD075 | CPD076 |
| 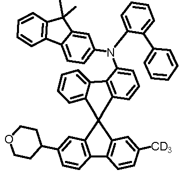 | 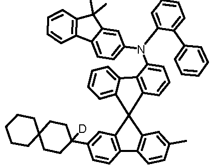 | 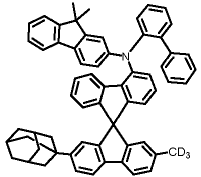 | 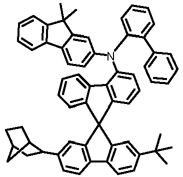 |
| CPD077 | CPD078 | CPD079 | CPD080 |
| 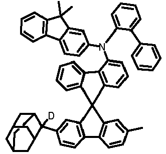 | 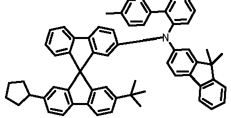 | 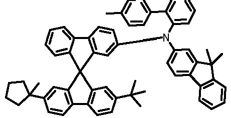 | 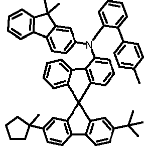 |
| CPD081 | CPD082 | CPD083 | CPD084 |
| 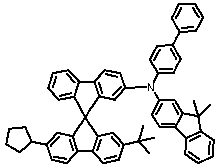 | 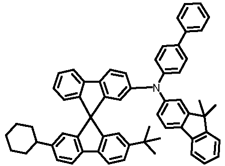 | 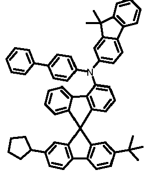 | 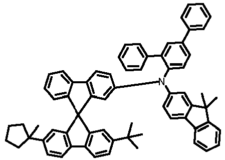 |
| CPD085 | CPD086 | CPD087 | CPD088 |
| 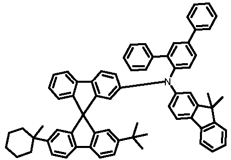 | 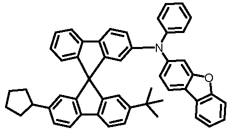 | 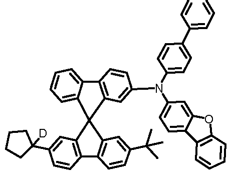 | 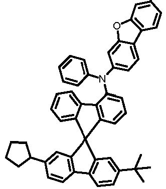 |
| CPD089 | CPD090 | CPD091 | CPD092 |
| 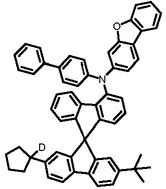 | 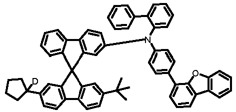 | 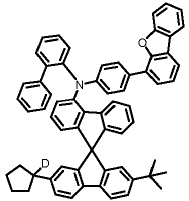 | 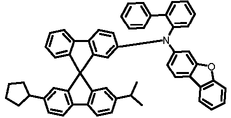 |
| CPD093 | CPD094 | CPD095 | CPD096 |
| 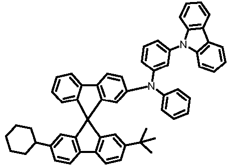 | 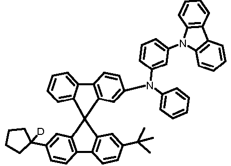 | 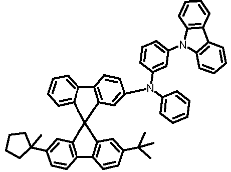 | 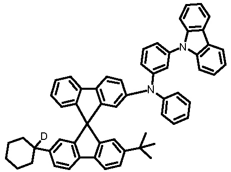 |
| CPD097 | CPD098 | CPD099 | CPD 100 |
| 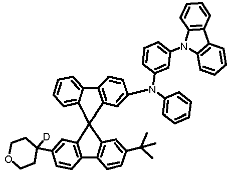 | 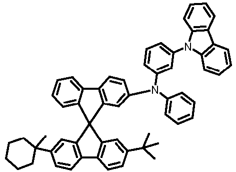 | 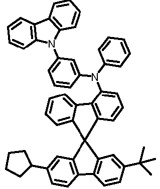 | 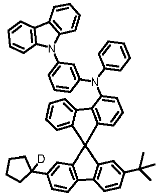 |
| CPD101 | CPD 102 | CPD 103 | CPD 104 |
| 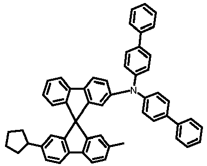 | 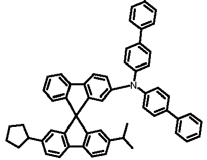 | 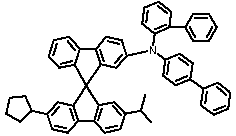 | 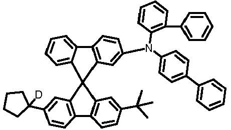 |
| CPD105 | CPD 106 | CPD 107 | CPD 108 |
| 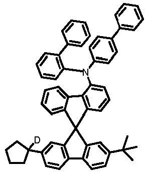 | 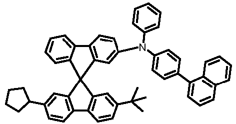 | 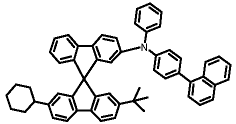 | 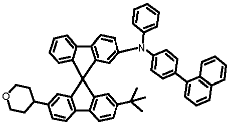 |
| CPD 109 | CPD110 | CPD111 | CPD112 |
| 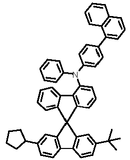 | 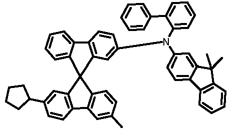 | 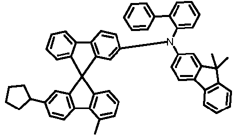 | 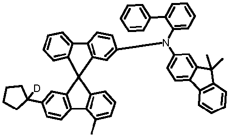 |
| CPD 113 | CPD 114 | CPD115 | CPD 116 |
| 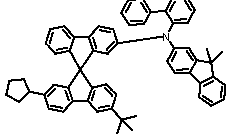 | 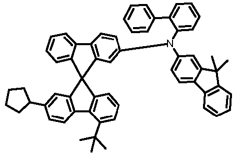 | 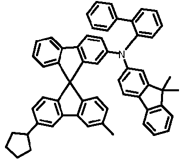 | 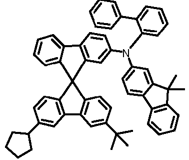 |
| CPD 117 | CPD118 | CPD 119 | CPD120 |
| 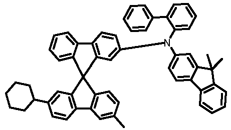 | 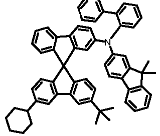 | 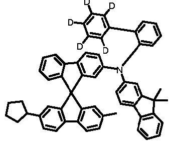 | 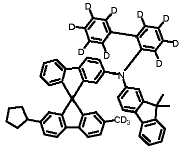 |
| CPD 121 | CPD 122 | CPD123 | CPD 124 |
| 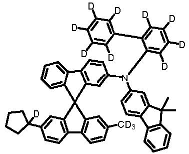 | 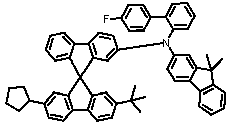 | 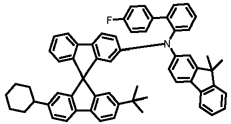 | 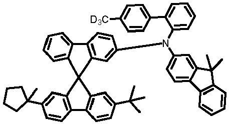 |
| CPD125 | CPD 126 | CPD127 | CPD128 |

13. An organic electroluminescent device, comprising the spiro compound according to any one of claims 1-12, wherein the spiro compound is a material for a hole injection layer and/or a hole transport layer of the organic electroluminescent device.
